# EUROPEAN PATENT APPLICATION

(11) **EP 4 011 371 A1**
(43) Date of publication of application: **15.06.2022**
(21) Application number: 20850185.8
(22) Date of filing: 06.08.2020
(51) Int. Cl.: A61K 31/164, A61K 38/01, A61K 31/375, A61P 31/12, A61P 31/04

(54) **PHARMACEUTICAL COMPOSITION PRODUCING SAFE AMOUNT OF NITRIC OXIDE IN BODY AND USE THEREOF**

(30) Priority: 06.08.2019 CN 201910719544
(71) Applicant: Lianyungang Jinkang Hexin Pharmaceutical Co. Ltd., Lianyungang, Jiangsu 222000 (CN)
(72) Inventor: CHENG, Yongzhi, Lianyungang, Jiangsu 222006 (CN); LIAN, Zenglin, Lianyungang, Jiangsu 222006 (CN); LIU, Kang, Lianyungang, Jiangsu 222006 (CN); GU, Rui, Lianyungang, Jiangxi 222006 (CN)
(74) Representative: Biggi, Cristina
(86) International application number: PCT/CN2020/107409
(87) International publication number: WO 2021/023263

(57) **Abstract**

A pharmaceutical composition for producing a safe amount of nitric oxide (NO) in vivo and use thereof. The pharmaceutical composition comprises the following components: an NO toxicity decreasing agent, an optional NO extender, and a nitric oxide synthase inducer. Provided is the pharmaceutical composition which has high versatility and extremely effectively treats pathogenic microorganism infections. A new medicinal activity of 5-methyltetrahydrofolic acid, NMN, and dehydroascorbic acid is found, which has a variety of active effects on an immune system caused by pathogen infection, and capable of being used for treating or preventing disease caused by virus infections and other pathogen infections.

## Description

### CROSS-REFERENCE TO RELATED APPLICATIONS

The present application claims the priority of a prior application of Patent Application No. 201910719544.6 filed with the State Intellectual Property Office of China on August 6, 2019, the invention title of which is "Safe Nitric Oxide Composition and Use Thereof", and the disclosure of the above prior application is incorporated in the present application by reference in its entirety.

### TECHNICAL FIELD

The present invention belongs to the field of medicine, and specifically relates to a pharmaceutical composition which can produce nitric oxide in an animal body, and can provide a safe and sufficient amount of nitric oxide for prevention and treatment of diseases.

### BACKGROUND

In the history of mankind, new viruses continue to appear, and known viruses continue to mutate. When faced with new infectious viruses, there are no corresponding specific antibodies in the human body, resulting in periodic-like large-scale infections. Several human influenza virus pandemics have taken away many lives. The H1N1 influenza virus broke out in the United States and Mexico in 2009, and the COVID-19 virus broke out globally in 2020. Different individuals were infected with the same influenza virus, but the results were different. Some patients lost their lives, while others had almost no symptoms. Although the virulence of the virus may vary, the immune status of the host is also important.

When it is necessary to prevent and treat influenza virus infection, antibodies are the best means, but influenza viruses are developing rapidly, and selective pressure of antibodies on seasonal influenza viruses promotes the emergence of escape mutants. These mutants can cause epidemics in communities immune to early strains, which is why seasonal flu vaccines need to be updated frequently. Unfortunately, the specificity of antibody response also laid a foundation for the emergence of a pandemic. In the last century, there have been many influenza virus or coronavirus pandemics, including, A(H1N1), A(H2N2), A(H3N2), A(H1N1), SARS new coronavirus and COVID-19, occurred in 1918, 1957, 1968, 2009, 2001 and 2020, respectively. What is interesting is that in the epidemic development of influenza virus infection, the severity of hosts varies greatly. Some scholars have proved that the difference in immune cells, especially T cell infection and activation, leads to differences in the ability to resist influenza viruses [Kelso, Anne. CD4+ T cells limit the damage in influenza[J]. Nature Medicine, 2012, 18(2):200-202.].

There is evidence that T cells can mediate cross-protective immunity. T cells cannot prevent virus infections, but they can perceive infected cells by recognizing viral protein (epitope) fragments complexed with human leukocyte antigen (HLA) molecules on the surface of infected epithelial cells or antigen-presenting cells. Since T cells preferentially see epitopes derived from conserved internal proteins of the virus, cross-protective immunity has been attributed to pre-existing cytotoxic CD8+ T cells. These cells will kill the virus-infected cells presenting these conserved epitopes, reducing the time and severity of pandemic virus infection due to lack of antibody protection.

Due to discovery of antibiotics, there are already very good clinical treatment methods for bacterial infections, but for viruses, there are currently no good treatment methods. At present, treatment drugs for viruses in human mainly include two categories, namely M2 ion channel blockers and neuraminidase inhibitors. The M2 ion channel blockers have overall virus resistance effects and nervous system side effects, which make their clinical use not ideal. Although the neuraminidase inhibitors can induce viruses, their effects are relatively weak. In recent years, there have been a large number of virus outbreaks, such as avian influenza virus, African swine fever virus, and atypical pneumonia virus. The toxicological consequences of these viruses are very serious. For patients or sick animals, doctors cannot come up with good treatment methods. Not only are there no good treatment methods for new viruses, but people are also helpless with many long-existing viruses, including dengue fever virus, HIV, and so on. The best way to treat the virus is prevention, that is, a vaccine, which achieves the effect of preventing the virus through the body's immune system. The above facts also show that for the treatment of viruses, the idea of developing drugs which directly kill or inhibit pathogens with antibiotics actually gets half the result with twice the effort.

It is necessary that antiviral drugs are developed with new ideas. Use of the human immune system to treat virus infections is an important direction, especially for NO and immune-related drugs, in order to achieve a versatile antiviral effect.

Nitric oxide is colorless and tasteless, soluble in water, alcohols, and fats. Before the 1980s, nitric oxide was just an ordinary and useless chemical gas. It was only known that nitric oxide existed in automobile exhaust and gaseous pollutants from certain chemical processes. In 27 years before 1980, it was discovered that endothelial cells produced a substance (called "an endothelium-derived relaxing factor"). The first experimental paper submitted by Ignarro in 1986 claimed that the endothelium-derived relaxing factor (EDRF) was nitric oxide. These results aroused people's great enthusiasm to pay attention to and study NO. NO quickly enters and exits cells, and conducts signals to regulate blood vessel expansion, nerve conduction, brain development, and even learning and memory. NO can strengthen immunity, kill some foreign microbes, lower blood pressure, and prevent stroke, heart disease, tumors, and Alzheimer's disease.

NO is reduced by Nicotinamide Adenine Dinucleotide Phosphate (NADPH) from L-arginine under the catalysis of nitric oxide synthase (NOS). The nitric oxide synthase can be divided into endothelial nitric oxide synthase (eNOS), inducible nitric oxide synthase (iNOS), and neural nitric oxide synthase (nNOS). They are respectively involved in the regulation of the cardiocerebral vascular system, immune regulation, and nervous system regulation in different tissue cells of a human body.

NO involved in immunity can be produced by multiple immune cells (dendritic cells, NK cells, macrophages, eosinophils and neutrophils). When iNOS is expressed, a large amount of NO can be produced, which acts as the body's active defense mechanism. There is evidence that NO can inhibit virus replication, and related mechanisms include reducing palmitoylation of viral spike proteins, inhibiting viral proteases, and hindering viral protein and nucleic acid synthesis.

Nitric oxide synthase is a dimer, which will uncouple under oxidizing conditions, resulting in the conversion of a reaction pathway originally synthesizing NO to production of O2-, NO3- (PON) and other reactive oxygen species (ROS). NO itself can also react with the ROS to produce reactive nitrogen species (RNS).

NO rapidly reacts with superoxide anions in vivo to produce peroxynitrous acid. Under acidic conditions, the peroxynitrite acid will quickly decompose to produce hydroxyl radicals. The peroxynitrite acid is a highly oxidizing substance which can cause protein nitration and DNA strand breakage. Various reasons lead to the production of many oxidative free radicals in the organism, including both ROS and RNS. These free radicals disrupt the balance of the past to a considerable extent. Among these free radicals, the most influential one is peroxynitrite anions (PON), the production way of which is mainly the reaction of nitric oxide with superoxide anions.

Most of the effects of PON in the human body are negative, including but not limited to:
1. Oxidation: PON itself is a strong oxidant. Under acidic conditions, PON rapidly decomposes to produce nitrogen dioxide and hydroxyl radicals. Hydroxyl radicals are stronger oxidants, and can oxidatively degrade almost all organic substances. In a living body, PON can react with the iron/sulfur centers of multiple enzymes, proteins and cytokines, sulfhydryl groups, lipids, etc., causing oxidative damage, and causing cell function damage and apoptosis. PON can also reduce the mechanism of glutathione to scavenge free radicals, causing a vicious circle. PON oxidation may cause various diseases, such as acute and chronic inflammation, sepsis, traumatic ischemia, arteriosclerosis, and nerve regeneration disorders.
2. Nitration: PON can react with tyrosine in proteins to produce nitrotyrosine, affecting the function of the proteins, and causing DNA breakage and other consequences.
3. Affecting energy metabolism: The activity of zymoprotein decreases under oxidation and nitration. For example, the activity of mitochondrial ATP synthase and aconitase is inhibited, resulting in a decrease in energy. PON is a strong activator of poly ADP-ribose synthase. Activation of this enzyme will initiate an ineffective repair cycle, causing quick depletion of an energy pool. Cell metabolism and membrane integrity are destroyed, leading to cell death.
4. Interfering with calcium transport: Sulfhydryl groups of Na+/Ca2+ exchange proteins are oxidized and dysfunction occurs, leading to calcium overload in the cell and causing dysfunction.

Of course, a tolerable dose of PON also shows positive effects, for example, resisting the harm of viruses, germs, pathogens, cancer cells and the like to the human body.

NO, the star molecule in 1992, is actually everywhere in living bodies. NO is a messenger of the immune system and plays an important role in regulating blood flow, nerve conduction, and brain development. NO can kill germs, viruses, pathogens, and cancer cells, and is a very important part of non-specific immunity. Foreign microbes or abnormal cells killed by NO can release a large amount of antigen substances after autolysis, and initiate specific immunity. NO can also cause the body to release many cytokines such as interleukin, interferon, tumor necrosis factors (TNF), and colony stimulating factors (CSF), to regulate the immune response

NO reacts with superoxide anions and other free radicals to form peroxynitrite (PON), which are extremely oxidative and has a special nitration ability. When accumulating to a certain extent, PON will cause inflammation and release cytokines affecting a pathological process. PON destroys protein functions through protein nitration, thereby breaking DNA strand, promoting virus variation, disrupting immune balance, awakening proto-oncogenes, and promoting cancer.

NO is involved in immune regulation. Acute inflammation is a complex but highly coordinated sequence of events involving molecular, cellular and physiological changes. Among them, if a host's response to the infection is unregulated, and an abnormal immune response is further caused, the resulting syndrome of organ dysfunction is called sepsis. Studies on the treatment of sepsis reflect the advancement of people's understanding of pathophysiology and host-microbe interactions. In the early days, people mainly paid attention to microbes and pathogenicity thereof. In the 1980s, with implementation of molecular cloning and sequencing of human inflammatory genes, the study on sepsis focused more on a host's response to invading pathogens.

According to The Third International Consensus Definitions for Sepsis in 2016, sepsis is defined as an organ dysfunction which threatens the host's life due to imbalance of the host's response to infection. Clinical manifestations of sepsis include fever, accelerated breathing, changes in consciousness and low blood pressure, and are accompanied by symptoms related to sepsis such as pneumonia caused by lung infection, kidney infection, and urinary tract infection.

Although human understanding of the origin and progress of sepsis has greatly improved, the mortality of sepsis is still very high. According to an article [Hotchkiss R S, Moldawer L L, Opal S M, et al. Sepsis and septic shock[J]. Nature reviews Disease primers, 2016, 2(1): 1-21.], preliminary inferences based on data from high-income countries indicate that 31.5 million cases of sepsis and 19.4 million cases of severe sepsis occur globally each year, and there may be 5.3 million deaths each year. In many cases, especially in patients with chronic diseases (such as cancer, congestive heart failure and chronic obstructive pulmonary disease), official death records usually report the underlying disease rather than the direct cause of death (sepsis), which may make the sepsis mortality be significantly underestimated. These figures are only estimates because the related morbidity and sepsis mortality records in low- and middle-income countries are still scarce.

Inflammation is a host's defense response to pathogen invasion. Therefore, clinically preferred treatment for removing pathogens is to use antibiotics or antiviral drugs to reduce external stimulation of pathogen antigens. Once virus infectious diseases develop to the stage of immune disorders, severe inflammation will occur. Some treatments to stop inflammation or anti-inflammation will reduce the number of macrophages in an inflamed area, and a decision to improve immunity or reduce immune response is often difficult to implement. Common anti-inflammatory drugs include non-steroidal anti-inflammatory drugs, glucocorticoids, etc. When severe inflammation occurs, glucocorticoids are often used clinically, but for sepsis, the use of cortisol has no substantial benefit. According to a randomized controlled trial [Annane D, Cariou A, Maxime V, et al. Corticosteroid treatment and intensive insulin therapy for septic shock in adults: a randomized controlled trial[J]. Jama, 2010, 303(4): 341-348.], fludrocortisone did not reduce the mortality of patients with sepsis. The subsequent extraction analysis [Wang C, Sun J, Zheng J, et al. Low-dose hydrocortisone therapy attenuates septic shock in adult patients but does not reduce 28-day mortality: a meta-analysis of randomized controlled trials[J]. Anesthesia & Analgesia, 2014, 118(2): 346-357.] also showed that hydrocortisone could not reduce the mortality of severely infected patients or sepsis. At present, the use of steroids for severely infected patients is controversial in clinical practice.

In the past 20 years, people have been trying to make clear the relationship between vitamin C and sepsis. Patients with sepsis generally have very low serum vitamin C levels. It is believed that low vitamin C levels in critically ill patients are associated with vascular compression, kidney damage, multiple organ dysfunction, and increased mortality. Through studies on the mechanism of vitamin C, a variety of mechanisms that may have an effect on sepsis have been discovered, including anti-oxidation, anti-inflammation, microcirculation, anti-thrombosis, increasing adrenal sensitivity, promoting wound healing, etc. However, unlike expectations, clinical use of vitamin C has no significant effect. According to statistics of [Chang Xueni, Li Min, Zhang Zhengxin, et al. Meta analysis of efficacy of vitamin C in treatment of patients with sepsis and septic shock[J]. Chinese Journal of Critical Care Medicine (Electronic Edition), 2019, 012(001):37-41.], intravenous infusion of vitamin C cannot improve the mortality of patients with sepsis and septic shock.

5-methyltetrahydrofolic acid is the active form of folic acid in a human body, and it is not observed that 5-methyltetrahydrofolic acid has a direct antiviral effect. At present, the direct link between folic acid and viruses is mainly folate receptor α (FRα), which has been described as a factor which mediates viruses including Ebola into cells. 5-methyltetrahydrofolic acid has a direct antioxidant effect. It promotes the conversion of BH2 to BH4 through dihydrofolate reductase. It is well known that BH4 is an essential cofactor for eNOS. It has been proved that 5-methyltetrahydrofolic acid is beneficial to prevention and protection of cardiovascular diseases through promoting eNOS. However, there are few reports on the effect of the 5-methyltetrahydrofolic acid on iNOS and NO secreted by macrophages under the condition of activation of innate immune.

L-arginine is the precursor for endogenous synthesis of NO. Under the action of nitric oxide synthase, L-arginine will react to produce NO and L-citrulline. Although only a small part of L-arginine is metabolized in vivo in this way, in the case of acute inflammation, NO produced by iNOS of macrophages can greatly exceed a normal dose of the human body. L-arginine is a non-essential amino acid, and can be synthesized endogenously in the metabolic pathways of proline, glutamine or glutamate (in the process of systemic protein degradation). In the kidney, citrulline is converted into arginine through arginine succinate synthase and arginine succinate lyase. However, when endogenous synthesis of arginine is insufficient to meet metabolic needs of an organism, arginine is very important under different pathophysiological conditions.

### SUMMARY

The present invention finds that 5-methyltetrahydrofolic acid at a "pharmacological" concentration has a physiological activity, which is different from that at a low concentration as a "nutrition support", and a composition containing 5-methyltetrahydrofolic acid has an effect of treating virus infections. It is further discovered that 5-methyltetrahydrofolic acid has therapeutic effects on different pathogens, including bacteria, fungi, etc. The present invention also finds that the activity of dehydroascorbic acid and nicotinamide mononucleotide (NMN) is similar to that of 5-methyltetrahydrofolic acid.

Based on the foregoing findings, the present invention provides the following technical solutions:
Disclosed is a pharmaceutical composition for producing a safe amount of nitric oxide in an animal body, that is, controlling or reducing the proportion of RNS in vivo, and the composition enables the nitric oxide produced in vivo to reach a dose required for prevention and treatment of diseases.

The pharmaceutical composition of the present invention includes an NO toxicity decreasing agent and an optional NO extender. The NO toxicity decreasing agent is selected from antioxidant substances for scavenging peroxynitrous acid or salt thereof (PON) at a dose. Preferably, the toxicity decreasing agent does not inhibit expression of inducible nitric oxide synthase (iNOS) at a concentration of not less than 10 µmol/L, for example, does not inhibit expression of iNOS in macrophages induced by LSP.

The NO toxicity decreasing agent of the present invention is selected from antioxidant substances, which do not affect activation of iNOS and selectively quench peroxynitrite. For example, the NO toxicity decreasing agent is selected from one or more of the following substances: 5-methyltetrahydrofolic acid or salt thereof, dehydroascorbic acid, and NMN.

The NO extender of the present invention is selected from enzyme-producing NO substrates, and the enzyme-producing NO substrates are selected from L-arginine or salt thereof, citrulline or salt thereof, or arginine activator additive.

The pharmaceutical composition of the present invention includes 5-methyltetrahydrofolic acid or salt thereof and arginine or salt thereof. Further, the pharmaceutical composition may include phytohemagglutinin.

In the pharmaceutical composition of the present invention, a single dose of the 5-methyltetrahydrofolic acid is not less than 15 mg, and a single dose of the arginine is not less than 50 mg.

The present invention further provides use of the pharmaceutical composition for preparing drugs for preventing or treating diseases caused by pathogenic microorganism infections. Preferably, the pathogenic microorganism infection is virus infection.

According to the use of the pharmaceutical composition of the present invention, the pharmaceutical composition can increase the level of T cells in a virus-infected host, especially CD4 and CD8 T cells, and reduce expression of inflammatory factors, thereby being used for anti-viral infection.

According to the use of the pharmaceutical composition of the present invention, the virus is influenza virus, herpes virus, African swine fever virus, and coronavirus such as COVID-19.

According to the use of the pharmaceutical composition of the present invention, the composition is used for preparing drugs for preventing and treating sepsis and systemic inflammatory response syndrome caused by infection.

The pharmaceutical composition according to the present invention includes 5-methyltetrahydrofolic acid or salt thereof and vitamin C. Preferably, a mass ratio of the 5-methyltetrahydrofolate calcium to the vitamin C is 2:1 to 5:1, e.g., 3:1, 4:1.

The present invention further provides use of the pharmaceutical composition, for preparing drugs for treating systemic inflammatory response syndrome and sepsis caused by non-infectious factors.

According to the use of the pharmaceutical composition of the present invention, the sepsis is caused by *Staphylococcus aureus, Streptococcus pneumoniae, Pseudomonas aeruginosa,* and influenza virus infection.

The pharmaceutical composition according to the present invention can be prepared from active components and pharmaceutically acceptable auxiliary materials, e.g., the pharmaceutical preparation is selected from tablets, capsules, granules, injections, topical ointments or sprays.

The pharmaceutical composition according to the present invention is an immune adjuvant.

In the present invention, a safe amount of nitric oxide means that the proportion of nitric oxide converted into toxic free radicals and RNS represented by peroxynitrous acid is controllable, and safety requirements in using nitric oxide to prevent and treat diseases can be met. These free radicals severely affect the metabolism of substances and energy in vivo, affect and even destroy the functions of cells and tissues, significantly increase the chance of gene mutations, and are also the cause of many diseases.

The composition of the present invention controls toxic free radicals and effectively increases the amount of nitric oxide produced to meet the requirements for preventing and treating diseases.

In the present invention, the pharmaceutical composition capable of producing a safe amount of nitric oxide has use potential in treatment of various diseases. The composition of the present invention can promote proliferation and activation of T cells, increase the level of CD4 and CD8 cells in a host in the course of infection, block apoptosis of CD4 and CD8 T cells, significantly increase the survival rate of the host, and improve inflammatory response in the course of infection.

The present invention administers a composition containing 5-methyltetrahydrofolic acid and arginine to mice infected with influenza virus, and obtains a large proportion of healing results with the course of disease significantly shortened.

The main function of folic acid is a carbon transmitter, and folic acid participates in DNA methylation, synthesis of purine and thymine, and further synthesis of DNA and RNA. Viruses replicate in host cells in large amounts with the structure of DNA or RNA. An adequate supply of folic acid should be conducive to the replication and transmission of the virus. An experimental result is surprising. 5-methyltetrahydrofolic acid combined with a nitric oxide extender inhibited the virus. The present invention proposes use of the composition of 5-methyltetrahydrofolic acid and arginine in microbial infections, especially viral infections for the first time.

iNOS is a key enzyme that produces NO in an immune system. It is known in the prior art that oxidation of iNOS will cause uncoupling of dimers, and a reaction pathway for producing NO is converted into a reaction pathway for producing free radicals and RNS. 5-methyltetrahydrofolic acid is an endogenous antioxidant, and can activate NADPH to achieve a good antioxidant effect and exert a direct antioxidant effect. The composition of the present invention can enable the body with pathogen infection to produce NO while avoiding production of free radicals unfavorable to the body, including reactive oxygen species (ROS) and reactive nitrogen species (RNS). The present invention has verified that antioxidants including 5-methyltetrahydrofolic acid or salt thereof, dehydroascorbic acid, and NMN can scavenge peroxynitrite without affecting the function of iNOS expression.

The present invention provides a method for producing sufficient nitric oxide in vivo. The composition of the present invention inhibits production of peroxynitrite without inhibiting but inducing increase in the activity of nitric oxide synthase, and further increases an enzyme-producing nitric oxide substrate arginine and precursors thereof, so that sufficient nitric oxide is produced in vivo.

In the present invention, the concept of sufficient amount refers to the minimum dose of nitric oxide reaching or exceeding that required for preventing and treating diseases.

The present invention provides a systematic solution for providing sufficient nitric oxide, which can be selected and optimized as required. To increase the output of nitric oxide, a nitric oxide synthase inducer, such as phytohemagglutinin, can also be used in the composition. Phytohemagglutinin (PHA) is a mitogen and an efficient and safe nitric oxide synthase inducer, and can be produced on a large scale by a technology of extraction from legumes. Another objective of the present invention is to provide multiple uses of the safe nitric oxide composition.

The active component in the composition of the present invention includes 5-methyltetrahydrofolic acid or salt thereof. The salt is selected from but not limited to calcium salt, arginine salt, glucosamine salt, and sodium salt.

In a preferred embodiment, the amount of 5-methyltetrahydrofolic acid or salt thereof in a single dose of composition of the present invention is 15 mg or more, preferably 25 mg or more, and more preferably 50-1000 mg.

In one embodiment, the composition includes 5-methyltetrahydrofolic acid or salt thereof, or dehydroascorbic acid, or NMN, and arginine; and the amount of 5-methyltetrahydrofolic acid or salt thereof in a single dose of composition is 15 mg (equivalent to 5-methyltetrahydrofolic acid) or more, preferably 25 mg or more, more preferably 50-1000 mg, and further more preferably 50-500 mg. For example, the amount of arginine is 50-5000 mg, preferably 100-1000 mg.

In one embodiment, the composition includes 5-methyltetrahydrofolic acid or salt thereof, arginine and phytohemagglutinin (PHA). The amount of 5-methyltetrahydrofolic acid or salt thereof per unit dose of composition is 15 mg or more, preferably 25 mg or more, more preferably 50-1000 mg, and further more preferably 50-500 mg; the amount of arginine per unit dose of composition is 50-5000 mg, preferably 100-1000 mg; and the amount of phytohemagglutinin per unit dose of composition is 10-500 mg, preferably 20-100 mg.

The pharmaceutical preparation can be selected from tablets, capsules, granules, injections, topical ointments or gas preparations.

### Detailed description of the invention

NO-induced stabilized and phosphorylated p53 levels of HIF-1α are reduced by ROS [Thomas DD, Ridnour LA, Espey MG, et al. Superoxide fluxes limit nitric oxide-induced signaling. J Biol Chem. 2006;281(36):25984-25993.]. In fact, addition of antioxidants has a protective effect on nitrosation signals [Edirisinghe I, Arunachalam G, Wong C, et al. Cigarette-smoke-induced oxidative/nitrosative stress impairs VEGF- and fluid-shear-stress-mediated signaling in endothelial cells [retracted in: Rahman I. Antioxid Redox Signal. 2013 Apr 2018(12):1535]. Antioxid Redox Signal. 2010;12(12):1355-1369.]. Therefore, NO levels and subsequent downstream signal transduction are regulated by ROS, which is also a factor in regulating redox signals.

Expression of iNOS requires simultaneous activation of STAT and NF-κB. NF-κB acts as a main switch of inflammation and is related to production of H₂O₂. NF-κB is regulated by redox. Most reducing agents or antioxidants have anti-inflammatory effects to some extent, inhibit the NF-κB pathway, and can inhibit expression of iNOS. In one embodiment, we compared the effects of different antioxidants on the expression of iNOS in macrophages induced by lipopolysaccharide (LPS). The results showed that 5-methyltetrahydrofolic acid, dehydroascorbic acid, BH4, glutathione, and NMN had almost no effect on the expression of iNOS at a concentration of 10 µmol/L. The reactivity of the above antioxidants with peroxynitrite is investigated, and the results show that 5-methyltetrahydrofolic acid, dehydroascorbic acid, and NMN all have a higher ability to scavenge peroxynitrite. It has been proven that under a hypoxic condition, the immune function of lymphocytes is suppressed and the rate of apoptosis increases. Due to lack of ROS, synthesis of iNOS is hindered, combination of iNOS and α-actinin4 is destroyed, and iNOS is prevented from attaching to the actin cytoskeleton. Therefore, antioxidants may cause down-regulation of iNOS. However, the present invention finds that the following antioxidants, namely 5-methyltetrahydrofolic acid, dehydroascorbic acid, and NMN, have unique properties, which do not reduce the expression of iNOS at a certain concentration, but have a better ability to scavenge peroxynitrite. The above antioxidants all have the ability to not reduce immune response after an antigen activates the immunity, especially not to negatively affect the expression of iNOS in the course of infection, and also reduce production of peroxynitrite. NO has an effect of inhibiting cell apoptosis. NO inhibits caspases-8, caspases-9 or caspases-3 through S-nitrosylation, while peroxynitrite promotes cell apoptosis through DNA damage and up-regulation of p53.

NO has direct and indirect effects on infectious microorganisms. NO can directly destroy the enzyme structure of pathogenic microorganisms, especially [Fe-S] clusters. In virus infection, expression of NO can inhibit the enzyme activity of the virus and inhibit replication of the virus. Direct toxicity of NO, especially the extracellular antiviral activity, has been fully demonstrated, but indirect effect of NO on regulation of the immune function is much more complicated. Studies have proven that iNOS-deficient mice infected with influenza virus have almost no histopathological evidence of pneumonia. Therefore, the scholar believes that iNOS of a host may contribute more to pneumonia than virus replication [Karupiah G, Chen JH, Mahalingam S, Nathan CF, MacMicking JD. Rapid interferon gamma-dependent clearance of influenza A virus and protection from consolidating pneumonitis in nitric oxide synthase 2-deficient mice. J Exp Med. 1998;188(8):1541-1546.]. In endotoxemia, the results of a preclinical model treated with an iNOS inhibitor in early stages are disappointing [Hauser B, Bracht H, Matejovic M, et al. Nitric oxide synthase inhibition in sepsis? Lessons learned from large-animal studies[J]. Anesthesia & Analgesia, 2005, 101(2): 488-498.]. The beneficial and harmful effects have been described so far, and people wonder whether NO is a positive or negative factor of infection.

It has been observed that exogenous NO inhibits proliferation of T lymphocytes, and exogenous NO (that is, NO is not produced by T cells) inhibits proliferation or even causes death of T cells [Bogdan C. Regulation of lymphocytes by nitric oxide[J]. Methods Mol Biol, 2011, 677:375-393.]. Mice lacking an important antioxidant mechanism (i.e., S-nitrosoglutathione reductase (GSNOR)) show a significant lack of T and B cells in the periphery due to excessive S-nitrosylation and lymphocyte apoptosis. Moreover, a small number of NO branch T cell subsets, especially Th1 cells and negative regulatory T cell populations of FoxP3, can effectively inhibit Th17 cell differentiation. In addition, recent studies have shown that exogenous NO also regulates Th9 and Th17 cells.

In an embodiment of the present invention, it finds that in a cell culture medium, 5-methyltetrahydrofolic acid at a concentration of 15.625 µm hardly affects secretion of NO in macrophages. More interestingly, when no LPS stimulation is provided, 5-methyltetrahydrofolic acid is found to promote the secretion of NO at a low concentration.

Combined use of the NO toxicity decreasing agent and the NO extender selected in the present invention shows that the activity of CD4+ T cell proliferation after antigenic stimulation can be significantly increased. Existing studies have shown that virus clearance is mediated by antigen-specific CD8+ effector T cells, and memory CD4+ T cells play an important role in maintaining memory response of CD8+ T and B cells [Stambas J, Guillonneau C, Kedzierska K, et al. Killer T cells in influenza[J]. Pharmacology & therapeutics, 2008, 120(2): 186-196.]. In addition, recent studies have shown that both CD4+ and CD8+ T cells are related to control of pneumonia, and limit excessive tissue damage through production of interleukin-10. Therefore, the pharmaceutical composition containing the NO toxicity decreasing agent and the NO extender of the present invention can be used in virus clearance and anti-inflammatory treatment.

In the present invention, as an extender of NO, arginine produces unexpected antiviral and sepsis treatment effects when used in combination with 5-methyltetrahydrofolic acid. In one embodiment, the composition of the present invention can significantly stimulate proliferation of T cells in the thymus and spleen of mice. The administration of the combination of arginine and 5-methyltetrahydrofolic acid can significantly increase proliferation of CD4 cells compared to addition of only arginine, which indicates that the composition can increase the proliferation ability of effector CD4+ T cells. As mentioned in the background art, the number of virus-specific memory CD4⁺ T cells can predict the severity of human infection with influenza virus, and the number of the virus-specific T cells is inversely proportional to the severity of disease. Accordingly, the composition of the present invention has a potential to treat influenza virus infection and can reduce the severity of disease. It has been known before that peroxynitrite affects immune response of cells. Studies have supported that peroxynitrite prevents the feedback ability of inhibiting inflammation and repairing, which can easily cause immune disorder of a host during infection. The used composition can not only improve the immunity of the host, but also maintain a negative feedback mechanism of inflammation, so that the host can defend against infection, especially viral infection.

Most of the existing viral influenza medicines are used to relieve symptoms and relieve the pain of influenza, but they cannot reliably or significantly shorten the course of the disease. The pharmaceutical composition of the present invention has a subversive effect on the treatment of influenza: the composition takes effect quickly. According to the follow-up visit results of more than 40 trial users, influenza symptoms generally disappeared within 48 hours after the composition is taken. Although no double-blind controlled clinical trials are conducted, the related feedback results of trial use of the composition are also beyond expectations.

Further, the present invention verifies the anti-viral infection effect of the composition in an animal model, and the results show that the composition can protect the immune function of mice, alleviate the pathological state of an influenza virus infected lung, and alleviate lung tissue damage. The composition of 5-methyltetrahydrofolic acid and arginine can significantly reduce the level of inflammatory factors caused by infection, and significantly reduce the virus titer in lungs 5 days after infection, which suggests that the composition has a certain antiviral effect. In addition, the use of the composition can significantly increase the levels of CD4⁺ and CD8⁺ T cells in the spleen and thymus of infected mice, which suggests that although the composition reduces the inflammatory factors, it does not reduce the immunity of the host. The results of lung tissue sections show that the composition can reduce lung tissue damage and inflammation, and shows excellent curative effects in a host model for influenza viruses.

Recent studies have shown that NO can promote immunological synapse (IS) signals mediated by T cell receptors (TCR) [Garcia-Ortiz A, Martin-Cofreces N B, Ibiza S, et al. eNOS S-nitrosylates β-actin on Cys374 and regulates PKC-θ at the immune synapse by impairing actin binding to profilin-l[J]. PLoS biology, 2017, 15(4): e2000653.]. IS is very important for regulating T cell activation, secretion and intercellular immune signal communication, which is also the possible reason why the composition can significantly increase the number of T cells.

In one embodiment of the present invention, the composition is used in treatment of pigs infected with African swine fever virus, excellent effects are achieved, and the survival rate of the pigs infected with African swine fever virus is significantly improved, which further proves the antiviral potential of the composition.

In addition, the present invention finds that the composition of the present invention can significantly protect the survival of a host in a high-dose virus challenge experiment, which shows a certain potential for the treatment of sepsis.

In the past three decades, more than 100 phase II and phase III clinical trials have been conducted to test various new drugs and therapeutic interventions in the hope of improving prognosis of patients with severe sepsis and septic shock. All these efforts ultimately failed to produce a new drug that can reduce organ failure and improve the survival rate of patients with sepsis [Artenstein AW, Higgins TL, Opal SM. Sepsis and scientific revolutions. Crit Care Med. 2013;41(12):2770-2772.]. All these studies use a single drug with specific molecules or pathways. Because very complex immune metabolic pathways and thousands of possible targets are involved, it is not easy to screen drugs with this idea.

Supplementation of exogenous arginine is controversial in treatment of sepsis. Since it was previously believed that NO-mediated peroxidation plays an important role in pathological development of sepsis, some people hypothesized that a pharmaceutical blocker in the course of NO production is a feasible strategy for the treatment of sepsis, so an NOS synthase inhibitor was developed. However, reviewing clinical results, therapies related to suppression of NOS are generally of no benefit. Moreover, the level of arginine in patients with sepsis decreases, but increasing an endogenous donor of NO may cause harmful effects of enhanced oxidative stress. Combined use of 5-methyltetrahydrofolic acid and arginine in the composition of the present invention has achieved unexpected excellent curative effects in preclinical animal models.

The present invention finds that 5-methyltetrahydrofolic acid can significantly reduce the mortality of LPS-induced sepsis mice, which suggests that 5-methyltetrahydrofolic acid may be beneficial to treatment of sepsis caused by severe allergies.

The present invention finds that the composition of 5-methyltetrahydrofolic acid and arginine can significantly reduce the mortality of sepsis mice caused by infection with microorganisms (e.g., *Staphylococcus aureus*). Sepsis is a highly fatal disease characterized by extensive apoptosis-induced depletion of immune cells and subsequent immunosuppression. The present invention finds that the composition of 5-methyltetrahydrofolic acid and arginine can significantly increase the survival rate of a host, and block apoptosis of CD4 and CD8 T cells, and the curative effects of the composition are proved in sepsis models caused by a variety of bacteria and viruses.

It should be recognized that certain oxidative signals in the human body are beneficial to infectious diseases, and there is a delicate balance between protective oxidative signals and harmful effects of ROS. The antioxidant used in the composition of the present invention has unique properties, can alleviate symptoms caused by pathogenic microorganism infection, and simultaneously increases the level of NO, so that an NO-mediated pathogen killing effect overcomes an oxidative stress mechanism triggered by NO.

T cells do play an important role in cross protection. In the field of vaccines, inactivated virus vaccines often protect against certain viruses by inducing specific antibodies, but have little effect on enhancing response of cross-protective T cells. The composition of the present invention has an effect of enhancing immune response. In one embodiment, the use of the composition significantly increases the antibody level of an immunized animal inoculated with a rabies vaccine.

The present invention makes use of the immune system of human and animals, which is a natural and powerful antiviral tool. African swine fever is a severe infectious disease, with a fatality rate of 95%-100%, to which the existing technology is completely useless. However, the present invention shows a clear curative effect, which shows that the anti-viral infection ability of the composition exceeds expectations. [Oura, C. A L. In vivo depletion of CD8+ T lymphocytes abrogates protective immunity to African swine fever virus[J]. Journal of General Virology, 2005, 86(9):2445-2450.] reported an attenuated virus isolate OUR/T88/3. After the virus isolate was used as a vaccine, it was found that infection with the non-toxic ASFV isolate OUR/T88/3 can protect distant relative pigs from challenge of a Portuguese ASFV virulent isolate OUR/T88/1. However, pigs exposed to OUR/T88/3 and then depleted of CD8(+) lymphocytes are no longer completely immune to OUR/T88/1 challenge. The result indicates that CD8+ lymphocytes play an important role in protective immune response to ASFV infection.

In recent years, great progress has been made in understanding the role of NO in immunity. In addition to directly inhibiting pathogenic microorganisms, NO also extensively regulates the immune function. In addition to iNOS, eNOS must be considered as a source of immune conditioned NO. Therefore, supplementary understanding of multiple functions and target spots of NO makes us realize that inhibition or activation of NOS subtypes is difficult to apply in clinical practice. However, the results of preclinical animal models of the composition of the present invention are encouraging, which shows the potential of NO in antiviral and sepsis treatment.

The present invention proposes a concept of applying safe and sufficient amount of nitric oxide in anti-viral infection for the first time, and the used compositions synergistically exhibit a good effect. There are complex regulatory mechanisms in the immune system, and multiple signaling pathways or target spots have beneficial or harmful actions. There are tens of thousands of research papers on nitric oxide, but the conclusions of the research are inconsistent, contradictory, and difficult to sort out. Some researchers even introduced a concept of "yin and yang balance" in Eastern civilization to express a double-edged sword-like role of various target mechanisms [Burke A J, Sullivan F J, Giles F J, et al. The yin and yang of nitric oxide in cancer progression[J]. Carcinogenesis, 2013, 34(3): 503-512.]. The composition of the present invention from the overall perspective of immunity achieves very significant technical effects: 1. significantly increasing the level of immune cells in a host, or preventing apoptosis of the immune cells; 2. effectively reducing inflammatory factors and alleviating inflammatory damage; 3. regulating the immune function for antivirus to make the host better resist secondary infection; and 4. being favorable in safety of components in the composition.

Term explanation:
NO, nitric oxide;
NOS, nitric oxide synthase;
SNO, safe nitric oxide, referring to nitric oxide with a controllable content of peroxynitrous acid and other toxic free radicals, and capable of meeting safety requirements when used to treat and prevent diseases;
PON, peroxynitrous acid and salt thereof;
NO toxicity decreasing agent, used to reduce reducing substances produced by peroxynitrous acid and other toxic nitrogenous free radicals;
NO extender, a precursor substance for producing NO, divided into chemical substances capable of releasing NO in vivo, and arginine, arginine activator additive, and other substances producing enzyme-producing NO;
NO synthase inducer, a substance for inducing the production of NO synthase; and Folate: 6S-5-methyltetrahydrofolate calcium.

The salt in the present invention refers to a pharmaceutically acceptable salt.

### BRIEF DESCRIPTION OF THE DRAWINGS

FIG. 1 shows body weight change curves of groups in Embodiment 7;
FIG. 2 shows body temperature change curves of groups in Embodiment 7;
FIG. 3 shows food intake change curves of groups in Embodiment 7;
FIG. 4 shows water intake change curves of groups in Embodiment 7;
FIG. 5 shows survival curves of groups in Embodiment 7;
FIG. 6 shows effects of various antioxidants on expression of iNOS in macrophages induced by LPS in Embodiment 8;
FIG. 7 shows scavenging actions of various antioxidants on peroxynitrite in Embodiment 9;
FIG. 8 shows effect of a composition on proliferation of CD4 T cells stimulated for three days in Embodiment 10;
FIG. 9 shows results of quantitative composition on proliferation of CD4 T cells stimulated for three days in Embodiment 10;
FIG. 10 shows results of detection of inflammatory factors in Embodiment 12;
FIG. 11 shows results of detection of inflammatory factors in Embodiment 12;
FIG. 12 shows results of detection of inflammatory factors in Embodiment 12;
FIG. 13 shows changes in splenic index and total number of immune cells in the spleen in Embodiment 13;
FIG. 14 shows changes in splenic index and total number of immune cells in the spleen in Embodiment 13;
FIG. 15 shows changes in splenic index and total number of immune cells in the spleen in Embodiment 13;
FIG. 16 shows changes in index and total number of immune cells in the thymus in Embodiment 13;
FIG. 17 is a diagram of lung tissue sections in Embodiment 13;
FIG. 18 shows changes in secretion of inflammatory factors in peripheral blood in Embodiment 13;
FIG. 19 shows virus titers of mice in groups in Embodiment 13;
FIG. 20 is a counting diagram of lymphocytes in the spleen of model mice with sepsis in Embodiment 17;
FIG. 21 shows changes in LYMP/NEUP of routine examination of venous blood from domestic pig ear caused by the composition in Embodiment 24.

### DETAILED DESCRIPTION

The foregoing and other features and advantages of the present invention is described and explained in more detail through the following descriptions of the embodiments of the present invention. It should be noted that the following embodiments are intended to exemplarily describe the technical solutions of the present invention, and are not intended to limit the protection scope of the present invention defined by the claims and equivalent schemes thereof.

Unless otherwise specified, the materials and reagents of this specification are all commercially available products, or may be prepared by a person skilled in the art.

### Embodiment 1 Capsules

6s-methyltetrahydrofolate calcium, 200 mg
Vitamin C, 200 mg
Filler, an appropriate amount
Binder, an appropriate amount
Disintegrant, an appropriate amount

### Embodiment 2 Capsules

6s-methyltetrahydrofolate calcium, 100 mg
Arginine, 400 mg
Filler, an appropriate amount
Binder, an appropriate amount
Disintegrant, an appropriate amount

### Embodiment 3 Tablets

6S-5-methyltetrahydrofolate calcium, 100 mg
Phytohemagglutinin, 50 mg
Arginine, 400 mg
Filler, an appropriate amount
Binder, an appropriate amount
Disintegrant, an appropriate amount

### Embodiment 4 Tablets

6S-5-methyltetrahydrofolate calcium, 200 mg
Vitamin C, 600 mg
Filler, an appropriate amount
Binder, an appropriate amount
Disintegrant, an appropriate amount

### Embodiment 5 Tablets

6S-5-methyltetrahydrofolate calcium, 400 mg
Sodium ascorbate, 100 mg
1,6-fructose diphosphate, 2 mg
Filler, an appropriate amount
Binder, an appropriate amount
Disintegrant, an appropriate amount

### Embodiment 6 Lyophilized powder for injection

6S-5-methyltetrahydrofolate calcium, 800 mg
Arginine, 3 g
Dissolved, filtered and lyophilized.

### Embodiment 7 Anti-influenza experiment in mice

Mice: 25 Balb/c mice, female, 6 weeks old, 15-17 g.
A: Administration group, infected and administered, 10 mice;
B: Model group, infected and not administered, 10 mice;
C: Normal group, not infected and not administered, 5 mice.

Infection method: Mice were anesthetized with 150 µl of 5% chloral hydrate injected intraperitoneally, and the mice were infected with PR8 influenza virus (1×10⁶ pfu/mouse) by nasal drip.

Administration method: 5-methyltetrahydrofolate calcium was prepared with distilled water to a concentration of 6 mg/ml, administered as per 200 µl/mouse, that was, 1.2 mg/mouse, by gavage 32 hours after the infection in this experiment.

Body weight, body temperature, water intake, and food intake were measured starting on the day of infection. The body weight, food intake and water intake of mice were measured once a day at a fixed time. The body temperature was measured twice a day within 3 days after infection at an interval of 12 hours, and starting from day 4 after infection, measured once a day at a fixed time. The experiment lasted to day 15 after infection, and at that time, the body weight of the mice basically recovered.

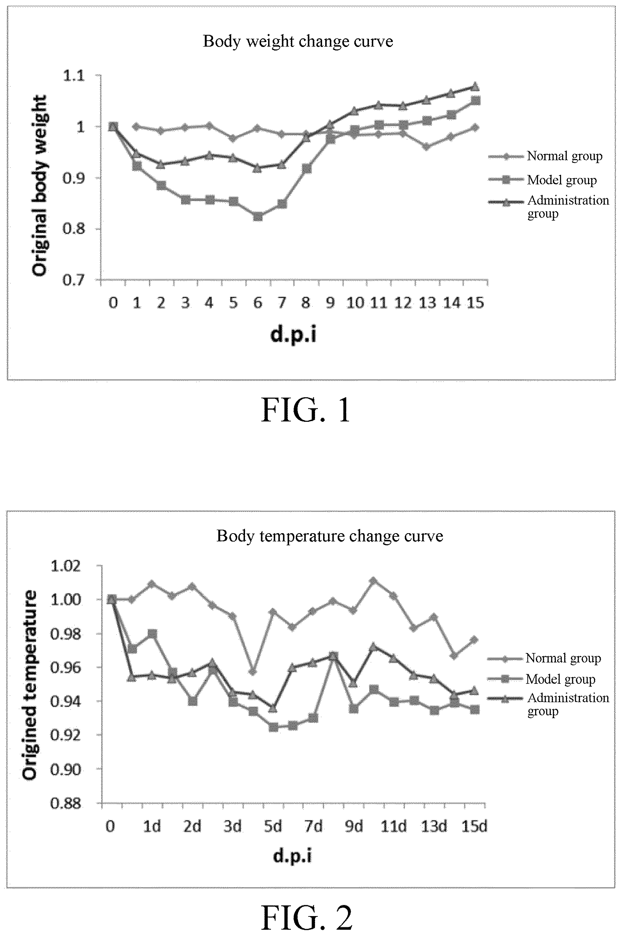

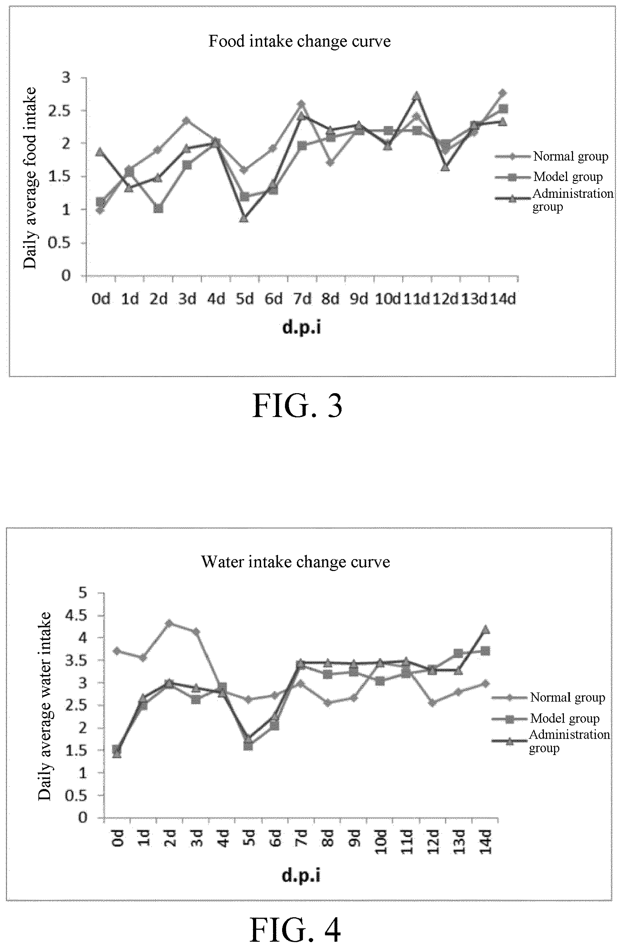

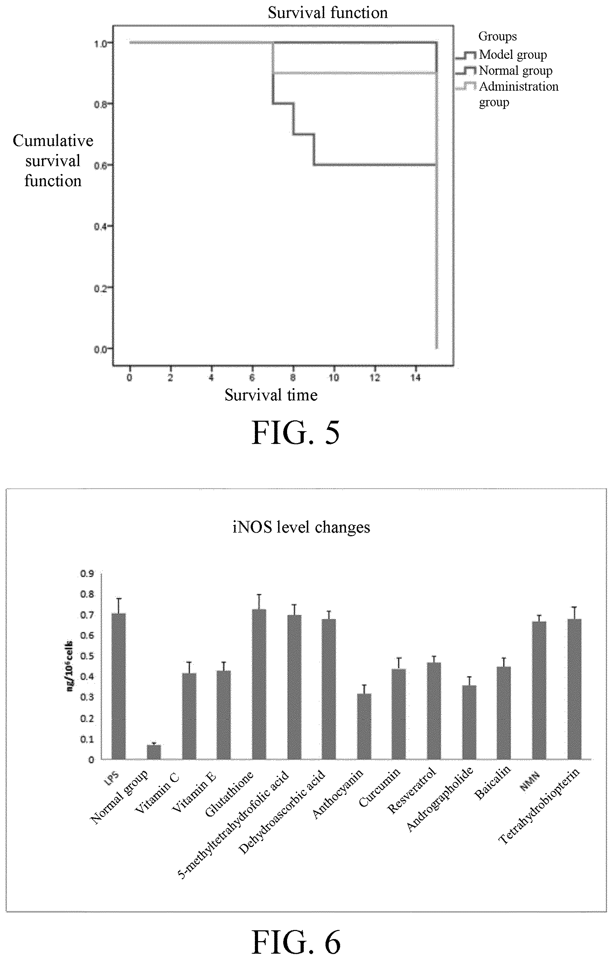

Results: The body weight change curve of each group is shown in FIG. 1, the body temperature change curve is shown in FIG. 2, the food intake change curve is shown in FIG. 3, the water intake change curve is shown in FIG. 4, and the survival curve is shown in FIG. 5. From the figures, the condition of the administration group was greatly improved. The ordinates in FIGs. 1-4 are relative ratios based on values as 1 on the first day.

### Embodiment 8 Screening of toxicity decreasing agents in the composition

### I. Experimental materials

1. Cell line: Macrophage RAW264.7.
2. Reagents: LPS (Sigma); iNOS detection kit (Stressgen); MTT (Biotopped).

### II. Experimental scheme

1. Cell culture:
   Mouse macrophages RAW264.7 were cultured in a Dulbecco's modified Eagle medium (DMEM) high glucose medium containing 10% FBS in a 37°C, 5% CO2 incubator.
2. Dosing treatment:
   The cell density was adjusted to 5×10⁴ cell/mL, and 100 µL of cell suspension was added per well of a 96-well plate, and cultured in a CO2 incubator for 24 h.

Induction of inflammation models based on LPS:
LPS induction: 40 µL of LPS was added per well (to a final concentration of 0.1 µg/mL);
Vitamin C group: Vitamin C and LPS were added per well (to a final concentration of 10 µmol/L of vitamin C, and 0.1 µg/mL of LPS);
Vitamin E group: Vitamin E and LPS were added per well (to a final concentration of 10 µmol/L of vitamin E, and 0.1 µg/mL of LPS);
Glutathione group: Glutathione and LPS were added per well (to a final concentration of 10 µmol/L of glutathione, and 0.1 µg/mL of LPS);
5-methyltetrahydrofolic acid group: 5-methyltetrahydrofolate calcium and LPS were added per well (to a final concentration of 10 µmol/L of 5-methyltetrahydrofolate calcium, and 0.1 µg/mL of LPS);
Dehydroascorbic acid group: Dehydroascorbic acid and LPS were added per well (to a final concentration of 10 µmol/L of dehydroascorbic acid, and 0.1 µg/mL of LPS);
Anthocyanin group: Anthocyanin and LPS were added per well (to a final concentration of 10 µmol/L of anthocyanin, and 0.1 µg/mL of LPS);
Curcumin group: Curcumin and LPS were added per well (to a final concentration of 10 µmol/L of curcumin, and 0.1 µg/mL of LPS);
Resveratrol group: Resveratrol and LPS were added per well (to a final concentration of 10 µmol/L of resveratrol, and 0.1 µg/mL of LPS);
Andrographolide group: Andrographolide and LPS were added per well (to a final concentration of 10 µmol/L of andrographolide, and 0.1 µg/mL of LPS);
Baicalin group: Baicalin and LPS were added per well (to a final concentration of 10 µmol/L of baicalin, and 0.1 µg/mL of LPS);
NMN group: NMN and LPS were added per well (to a final concentration of 10 µmol/L of NMN, and 0.1 µg/mL of LPS);
Tetrahydrobiopterin group: Tetrahydrobiopterin and LPS were added per well (to a final concentration of 10 µmol/L of tetrahydrobiopterin, and 0.1 µg/mL of LPS);
Normal group: 50 µL of complete medium was added per well.
All materials were mixed well and cultured for 24 h in a CO₂ incubator.

### 3. Detection of iNOS

The level of iNOS protein in macrophages was determined by ELISA using an anti-human iNOS polyclonal antibody (Stressgen). The number of macrophages was determined by using an automatic flow cytometer.

### 4. Results

The results are shown in FIG. 6. The results show that 5-methyltetrahydrofolic acid, glutathione, NMN, and tetrahydrobiopterin did not affect expression of iNOS at the concentration of 10 µmol/L.

### Embodiment 9 Comparison of different antioxidants in removing peroxynitrite

3-morpholino-sydnonimine (SIN-1) as a peroxynitrite donor was added to 15 test tubes at a concentration of 1 µmol/L. 5-methyltetrahydrofolate calcium was added to test tubes containing the SIN-1 solution to the final concentrations of 1 µmol/L, 10 µmol/L, and 100 µmol/L respectively; dehydroascorbic acid was added to test tubes containing the SIN-1 solution to the final concentrations of 1 µmol/L, 10 µmol/L, and 100 µmol/L respectively; glutathione was added to test tubes containing the SIN-1 solution to the final concentrations of 1 µmol/L, 10 µmol/L, and 100 µmοl/L respectively; NMN was added to test tubes containing the SIN-1 solution to the final concentrations of 1 µmol/L, 10 µmol/L, and 100 µmol/L respectively; and tetrahydrobiopterin was added to test tubes containing the SIN-1 solution to the final concentrations of 1 µmol/L, 10 µmol/L, and 100 µmol/L respectively. The concentration of peroxynitrite was determined by spectrophotometry at a detection wavelength of 302 nm.

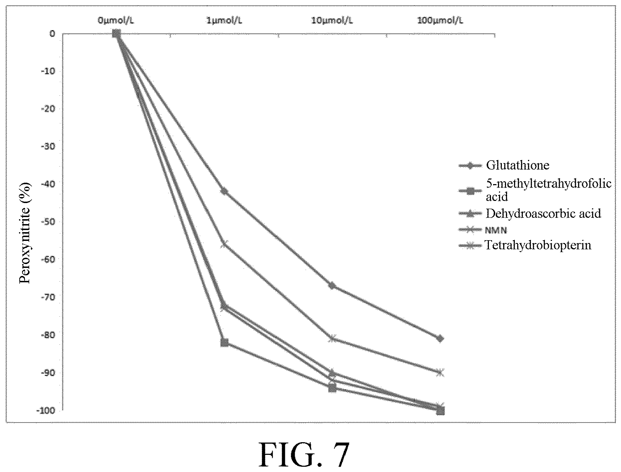

The results as shown in FIG. 7 show that dehydroascorbic acid, 5-methyltetrahydrofolic acid, and NMN all have excellent peroxynitrite scavenging effects.

### Embodiment 10 Experiment of T cell proliferation and differentiation under the intervention of the composition

Mice were sacrificed by cervical dislocation. The spleen and lymph nodes of the mice were aseptically separated and placed in a Hank's solution. CD4 T cells were purified from the spleen and lymph nodes of the mice using immune microspheres (CD4+ cell extraction kit; Miltenyi Biotec, USA). 4 µg/mL mouse CD3 monoclonal antibodies and 2 µg/mL anti-CD28 (Biolegend) were added to a 96-well plate, and DMEM (without L-arginine), trace penicillin, glycine, and 10% fetal bovine serum were added.

Composition A group: in a cell culture solution, 5-methyltetrahydrofolate calcium was added to a final concentration of 10 µmol/L, and arginine was added to a final concentration of 40 µmol/L; Composition B group: in a cell culture solution, dehydroascorbic acid was added to a final concentration of 10 µmol/L, and arginine was added to a final concentration of 40 µmol/L; Composition C group: in a cell culture solution, NMN was added to a final concentration of 10 µmol/L, and arginine was added to a final concentration of 40 µmol/L; Arginine group: in a cell culture solution, arginine was added to a final concentration of 40 µmol/L; and Blank group: the initial cell culture solution (without L-arginine) was used.

Purified T cells were stained with the Cell Violet Trace Proliferation kit (Invitrogen) and cultured for three days, and then analyzed by flow cytometry to determine proliferation.

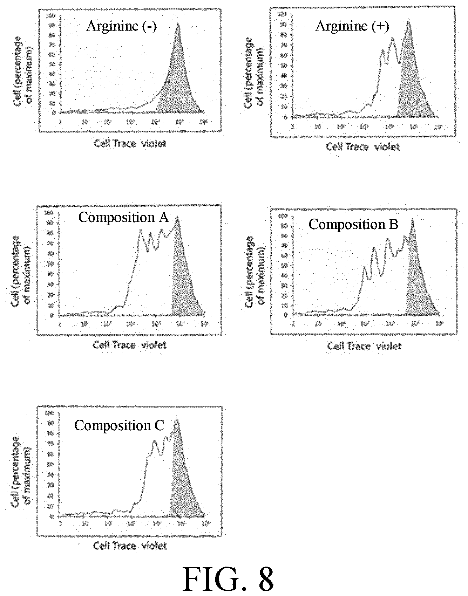

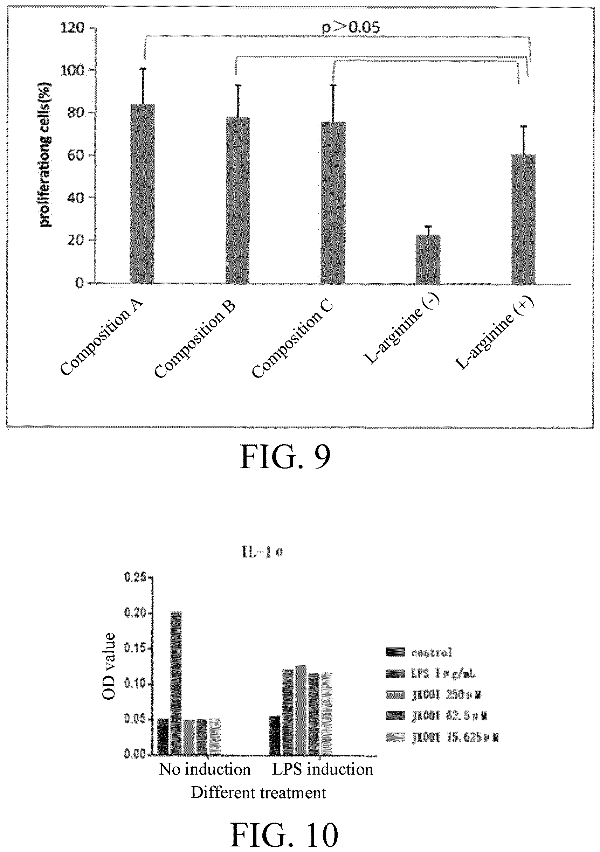

The results are shown in FIGs. 8 and 9. The results show that the selected compositions could increase proliferation of stimulated CD4 cells to a certain extent, and showed an ability to improve the cellular immunity of an infected host.

### Embodiment 11 Preliminary clinical experiment of trial use of the composition in influenza patients

Folate (6S-5-methyltetrahydrofolate calcium) capsules (400 mg/capsule) were prepared and given to influenza patients for trial use. Table 1 records the disappearance time (in hours) of symptoms after medication. The situation in which symptoms other than tonsillitis and sore throat disappeared was defined as basic rehabilitation; and the situation in which all symptoms including tonsillitis and sore throat disappeared was defined as complete rehabilitation.

**Table 1 Clinical statistics of influenza patients (disappearance time of symptoms, hours)**

| Patient No. | Headache | Nasal stuffiness | Running nose | Sneezing | Low fever | Fatigue | Dry and itchy throat | Intolerance of cold and cold limbs | Muscular soreness | Inappetence | Dizziness and fullness in head | Cough | Sore throat | Basic rehabilitation | Complete rehabilitation |
|---|---|---|---|---|---|---|---|---|---|---|---|---|---|---|---|
| A01 | | | 33 | | | 33 | | | | | | 33 | 33 | 33 | 33 |
| A02 | | 9 | 11 | | | 35 | | | 16 | | | | | 35 | 35 |
| A03 | 13 | | 13 | | | | | | 13 | | | | | 13 | 13 |
| A04 | 12 | | | | | | | | | | | | 12 | 12 | 12 |
| A05 | | 17 | | | | | | | | | | 17 | | 17 | 17 |
| A06 | 32 | 32 | 32 | 32 | | | | | 32 | | | | 32 | 32 | 32 |
| A07 | 8 | 8 | 8 | | | 8 | | | | | | | | 8 | 8 |
| A08 | 9 | 43 | 9 | 9 | | | | | 9 | | | 29 | 29 | 43 | 43 |
| A09 | 12 | 12 | | 18 | | 12 | | | | | | | 36 | 18 | 36 |
| A10 | | 20 | 20 | 34 | | | | | | | | | 36 | 34 | 36 |
| A11 | | 15 | 15 | 15 | | 15 | 15 | | 15 | | 48 | | | 15 | 48 |
| A12 | | | 11 | 9 | | | | | | 13 | 9 | | | 11 | 13 |
| A13 | 8 | 56 | 12 | 12 | | 8 | | | | | | | | 56 | 56 |
| A14 | 8 | 32 | 12 | 8 | | 8 | | | | | | 46 | 46 | 32 | 46 |
| A15 | | 33 | 33 | 33 | | | | | | | | 33 | 33 | 33 | 33 |
| A16 | 12 | 12 | 12 | 12 | | 12 | | | | | | 44 | 12 | 12 | 44 |
| A17 | | 24 | | | | 10 | | | | | | 46 | 32 | 24 | 46 |
| A18 | 10 | | | | 10 | 10 | | 10 | 10 | | | 13 | 10 | 10 | 13 |
| A19 | 10 | 10 | 46 | | | 10 | | | 10 | | | | | 46 | 46 |
| A20 | | 15 | | 10 | | 10 | | | | | | | 54 | 58 | 58 |
| A21 | 12 | 41 | 53 | 17 | | 12 | | | | | | 58 | 58 | 53 | 58 |
| A22 | 32 | | | 32 | | | | | | | | | 32 | 32 | 32 |
| A23 | | 12 | 17 | 12 | | | | | | | | | | 17 | 17 |
| A24 | 14 | | 14 | | | | | | 14 | | | | 14 | 14 | 14 |
| Mean | 13.7 | 23.0 | 20.6 | 18.1 | 10.0 | 14.1 | 15.0 | 10.0 | 14.9 | 13.0 | 28.5 | 35.7 | 29.4 | 27.4 | 32.9 |

Prescribed GK301 capsules (folate 300 mg, L-arginine 100 mg) were administered by influenza patients, and the results are listed in Table 2.

**Table 2 Clinical analysis of influenza patients (disappearance time of symptoms, hours)**

| Patient No. | Headache | Nasal stuffiness | Running nose | Sneezing | Low fever | Fatigue | Dry and itchy throat | Intolerance of cold and cold limbs | Muscular soreness | Inappetence | Nausea | Cough | Sore throat | Basic rehabilitation | Complete rehabilitation |
|---|---|---|---|---|---|---|---|---|---|---|---|---|---|---|---|
| B01 | | 35 | 11 | 35 | | | | | | | | | | 35 | 35 |
| B02 | 15 | 18 | 15 | 18 | | | | | 41 | | | | | 18 | 41 |
| B03 | 6.5 | | 6.5 | | | 6.5 | | | | | | 38 | | 6.5 | 37.5 |
| B04 | 9 | | 9 | | | | | | | | | | 9 | 9 | 9 |
| B05 | 13 | | | | | | | | | | | 36 | | 13 | 36 |
| B06 | | | 11 | | 11 | 11 | | | | | | | | 11 | 11 |
| B07 | 13 | | 13 | | | 13 | | | | | | 84 | | 13 | 84 |
| B08 | 13 | | | 24 | | 13 | | | | 13 | | | 48 | 13 | 48 |
| B09 | | 15 | | 15 | | 15 | | | | 15 | 15 | | | 15 | 15 |
| B10 | 9 | | 9 | 9 | | 9 | | | | | | | | 9 | 9 |
| B11 | 16 | | 22 | | | 36 | | | | | | 60 | | 36 | 60 |
| B12 | 9 | 9 | 9 | | | | 9 | | | | 9 | | 9 | 9 | 9 |
| B13 | | 9 | 9 | 9 | | 19 | | | 9 | | | 9 | 33 | 19 | 33 |
| B14 | 9 | | 9 | 9 | | | | | | | | | | 9 | 9 |
| B15 | 12 | 12 | 12 | 12 | 12 | 12 | | 12 | 12 | | | 12 | 36 | 12 | 36 |
| B16 | 35 | | 32 | | | 32 | | 32 | 32 | 32 | 32 | | 32 | 35 | 35 |
| B17 | | 8 | | 8 | | 8 | | | | | | | 32 | 8 | 32 |
| B18 | 23 | 23 | 23 | | 11 | 23 | | 23 | 23 | 23 | | 23 | | 23 | 23 |
| B19 | | 15 | 15 | | | 15 | | | | | | | 15 | 15.0 | 15.0 |
| B20 | 16 | 16 | 16 | | | | | | | 16 | | | | 16.0 | 16.0 |
| B21 | 12 | | | 12 | | 12 | | | | | | | | 12.0 | 12.0 |
| Mean time | 14.0 | 16.0 | 13.8 | 15.1 | 11.3 | 16.0 | 9.0 | 22.3 | 23.4 | 19.8 | 18.7 | 37.4 | 26.8 | 16.0 | 28.8 |

Compared with Table 1, addition of the arginine shortened the course of the disease. The anti-influenza effect of the composition was better than that of the 5-methyltetrahydrofolic acid alone. However, data in both Table 1 and Table 2 show that after 5-methyltetrahydrofolic acid was taken, the patients' influenza almost all healed within 2 days.

### Embodiment 12 Effects of 5-methyltetrahydrofolic acid on some inflammatory factors and NO secretion

### I. Experimental materials

1. Cell line: Macrophage RAW264.7.
2. Reagents: LPS (Sigma); MTT (Biotopped); Folate, namely, 5-methyltetrahydrofolate calcium (Lianyungang Jinkang Hexin Pharmaceutical Co., Ltd.); NO detection kit (Beyotime).

### II. Experimental scheme

1. Cell culture: Mouse macrophages RAW264.7 were cultured in a DMEM high glucose medium containing 10% FBS in a 37°C, 5% CO₂ incubator. Inflammatory factors (TNF-α, IL-1α, IL-6) in the supernatant were detected by ELISA.
2. Dosing treatment:
   1) The cell density was adjusted to 2×10⁵ cell/mL, and 100 µL of cell suspension was added per well of a 96-well plate, and cultured in a CO2 incubator for 24 h.
   2) Folate group: 50 µL of Folate was added per well (to final concentrations of 15.625, 62.5, and 250 µmol/L);
      LPS+Folate group: 50 µL of LPS was added per well (to a final concentration of 0.1 µg/mL), and after culturing in an incubator for 6 h, 10 µL of Folate was added per well (to final concentrations of 15.625, 62.5, and 250 µmol/L);
      LPS group: 50 µL of LPS was added (to a final concentration of 0.1 µg/mL);
      Normal group: 50 µL of complete medium was added per well.
   3) All materials were mixed well and cultured for 24 h in a CO2 incubator.

An absorbance value at 520 nm was expressed as mean±standard deviation. NO secretion rate=(OD sample well-OD blank well)/(OD normal well-OD blank well)×100%, and the NO secretion amount was calculated according to a standard curve.

### III. Experimental results

**Table 3 OD values measured by an NO detection kit at 520 nm, expressed as mean values (n=6)**

| | | | Lysate | | Supernatant | |
|---|---|---|---|---|---|---|
| Treatment | Groups | Concentration (µM) | OD 520 nm | NO secretion amount (µM) | OD 520 nm | NO secretion amount (µM) |
| No induction | Normal group | 0 | 0.0481 | 0.888 | 0.1076 | 1.657 |
| | LPS group | 1 µg/mL | 0.0566 | 1.95 | 0.395 | 42.714 |
| | Folate group | 15.625 | 0.0719 | 3.863 | 0.108 | 1.714 |
| 1 µg/mL LPS induction | Normal group | 0 | 0.0665 | 3.188 | 0.1052 | 1.314 |
| | LPS group | 1 µg/mL | 0.0538 | 1.6 | 0.3574 | 37.343 |
| | Folate group | 15.625 | 0.0548 | 1.725 | 0.3537 | 36.814 |

The results show that the 5-methyltetrahydrofolic acid at the concentration of 15.625 µmol/L did not inhibit expression of NO in macrophages induced by LPS.

The experimental results of the inflammatory factors are shown in FIG. 10, FIG. 11 and FIG. 12 of the specification.

The above results indicate that the 5-methyltetrahydrofolate calcium has no significant effect on expression of macrophages and inflammatory factors induced by LPS.

### Embodiment 13 Investigation of the early protective effect of the composition at different doses of one-time administration on influenza virus infected mice

### 1.1 Materials and methods

### 1.1.1 Mice

20 Balb/c mice (5 in each group), female, 6 weeks old, 15-17 g, purchased from Vital River Laboratory Animal Co., Ltd.

### 1.1.2 Drug preparation

A drug was dissolved with deionized water, prepared and used within 30 minutes.

### 1.1.3 Administration method

Group G1: Blank control group
Group G2: Model group
Group G3: Low-dose administration group (5-methyltetrahydrofolic acid: arginine=1:4, 0.173 g/kg)
Group G4: High-dose administration group (5-methyltetrahydrofolic acid: arginine=1:4, 0.346 g/kg)

The low-dose group and the high-dose group were administered by gavage. The model group was only modeled but not administered, and given an equal volume of deionized water. The blank control group was given an equal volume of deionized water. All groups were administered once.

### 1.1.4 Infection method

Mice were anesthetized with 150 µl of 5% chloral hydrate injected intraperitoneally, and the mice were infected with PR8 influenza virus (1×10⁶ pfu/mouse) by nasal drip.

### 1.1.5 Mouse treatment method

The body weight of the mice was measured on the day of infection, and the body weight of the mice was measured once a day at a fixed time. 3 days after infection, 100 µl of venous blood was taken from the orbit, and serum was prepared and frozen. Mice were sacrificed 5 days after infection, and the lungs, the thymus, the spleen and peripheral blood were collected.

Blood: Serum was prepared, part of which was tested for cytokines (external test), and the rest was frozen.

The lung tissue was divided into 2 parts, one part (the right lung lobe) was used to determine the virus titer, and the other part (the upper tip of the left lung lobe) was fixed, paraffin-embedded, sectioned and HE stained.

The spleen and thymus were subjected to weighing, photographing, cell counting and immune cell staining.

### 1.1.6 Index observation

Body weight changes: The body weight was measured every day. Sample keeping: The serum sample on day 5 was used to detect inflammatory factors (detected using Biolegend's LEGENDplex Mouse Inflammation Panel, external test). The lungs were used to measure the virus titer, make pathological sections of lung tissue, and detect changes in inflammatory factors in the lung tissue. The thymus was subjected to weighing, photographing, total thymus cell counting, and lymphocyte staining (CD4⁺, CD8⁺ T cells). The spleen was subjected to weighing, photographing, total splenic cell counting, and splenic lymphocyte staining analysis (surface staining, B cells, CD4⁺ T cells, CD8⁺ T cells, NK cells, NKT cells, monocytes, macrophages, dendritic cells, and neutrophils).

### 1.2 Experimental results

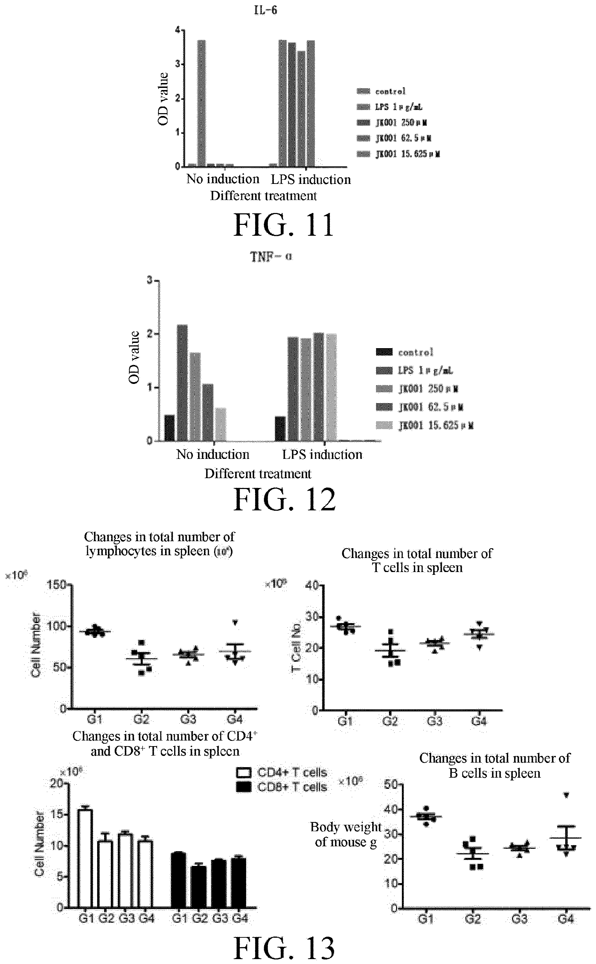

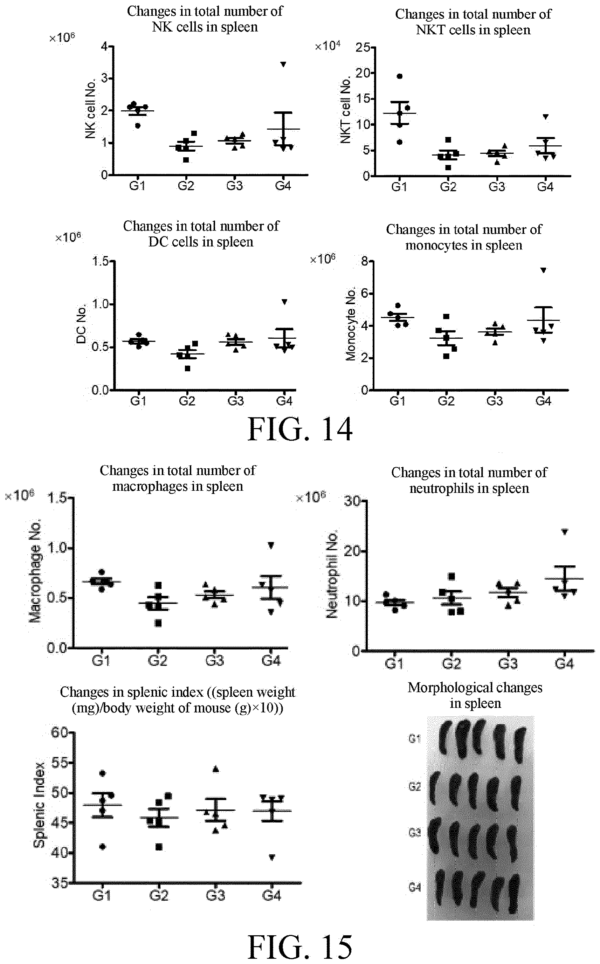

1.2.1 Changes in splenic index and the total number of immune cells in the spleen See FIG. 13, FIG. 14 and FIG.15 of the specification.

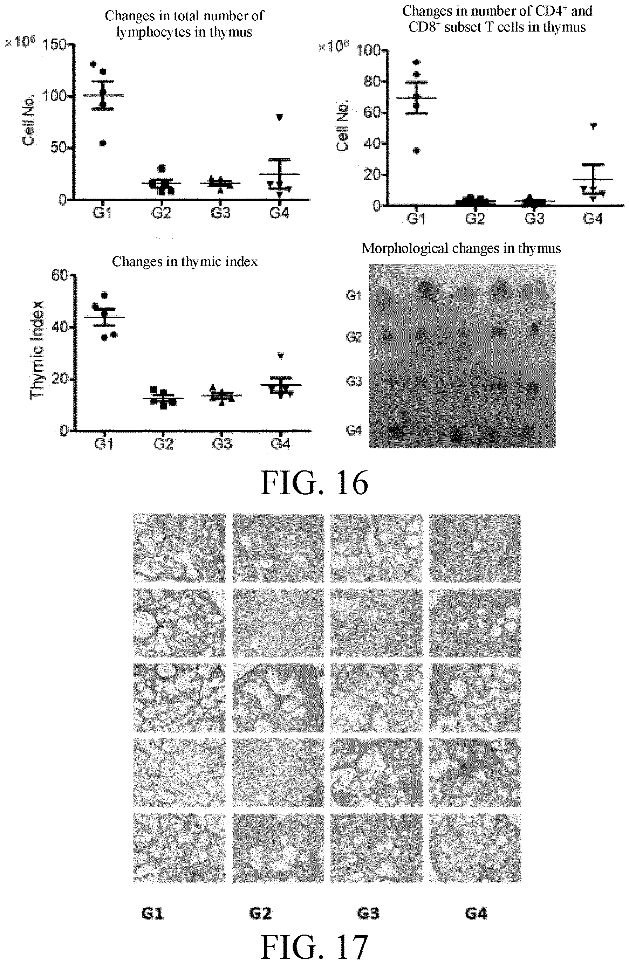

1.2.3 Changes in thymic index and the total number of immune cells See FIG. 16 of the specification.

### 1.2.2 Pathological changes in the lungs

See FIG. 17 of the specification.

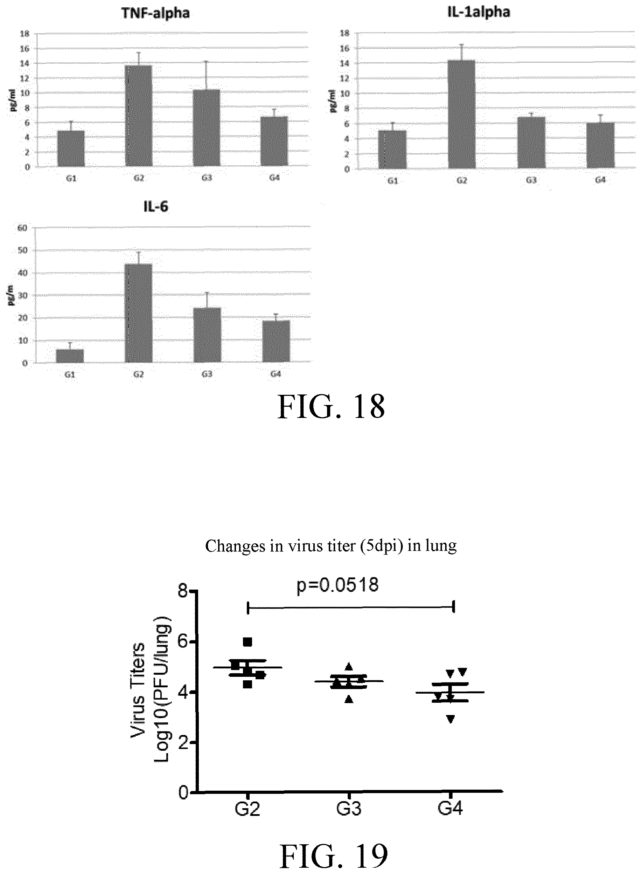

1.2.6 Changes in secretion of inflammatory factors in peripheral blood See FIG. 18 of the specification.

### 1.2.7 Changes in lung virus titer (5 dpi)

See FIG. 19 of the specification.

The results show that after infection with influenza virus, except the normal control group, in the other groups, the number of spleen lymphocytes of the mice decreased, and the thymuses were shrunk. The thymus changes of the high-dose group were smaller. The spleen and thymus are both immune organs and are related to the immune function of mice. Shrinkage of the thymus is one of the reasons for the weakened immune function, which suggests that the composition may help protect the immune organs and have a certain protective effect against the weakened immunity caused by viral infection. After the pathological sections of the lungs were stained with HE, the model group and the treatment groups showed similar lymphocyte infiltration and changes in lung tissue structure. The high-dose treatment group had slightly less lung tissue damage than the model group, which suggests that the composition helped to reduce a pathological state of the lungs in the early stage of influenza virus infection and reduce lung tissue damage. 5 days after infection with influenza virus, multiple cytokines in the peripheral blood of the model group significantly increased, and the administration group could significantly reduce secretion of inflammatory factors caused by the infection. It shows that drug intervention can effectively reduce the level of inflammatory factors, and may help to reduce the inflammatory factor storm and prevent lung damage caused by the inflammatory factor storm. Compared with the model group, lung virus titers in the treatment groups 5 days after infection showed a downward trend in varying degrees. Especially in the high-dose treatment group, the drop in virus titer compared with the model group approached a critical value of statistically significant difference, which suggests that the composition can inhibit replication of the virus in vivo by reducing the virus titer, and has a certain antiviral effect.

### Embodiment 14 Treatment of mice with herpes virus type I encephalitis by the composition

60 male Kunming mice weighing 14-18 g were used. Hela cells were attacked by HSV-1, and HSV-1 was cultured in the Hela cells for 48 hours. The virus was collected to determine the virus titer, and mice were inoculated with the virus with a mass fraction of 100TCID₅₀10⁻⁵. Mice were divided into a control group, a model group, a normal saline treatment group, an acyclovir treatment group (10 mg/kg), and a composition treatment group (5-methyltetrahydrofolic acid 14 mg/kg, arginine 50 mg/kg, and phytohemagglutinin 7 mg/kg). The control group was injected with 0.03 ml of sterile normal saline, and the model group and the treatment groups were injected with 0.03 ml of HSV-1 virus solution and then administered by gavage for 4 consecutive days. The death and other changes in each group were observed. After 7 days, 0.5 ml of blood was taken from the eyeballs, stored in a 35°C incubator for 2 h, and centrifuged at 1000 r/min for 5 min. The detection results of NO and 1L-1β are as follows:

**Table 4 Number of deaths and mortality of different groups**

| Groups | n | 48 h | D4 | D7 | Mortality |
|---|---|---|---|---|---|
| Control group | 15 | 0 | 0 | 0 | 0 |
| Model group | 15 | 2 | 5 | 8 | 100% |
| Acyclovir treatment group | 15 | 0 | 4 | 5 | 60% |
| Composition treatment group | 15 | 2 | 4 | 1 | 33% |

**Table 5 The levels of NO and 1L-1β in the serum of mice in the control group, model group, acyclovir group and nitric oxide composition group (x̅±s)**

| Groups | n | NO (µmol/L) | 1L-1β (mg/L) |
|---|---|---|---|
| Control group | 15 | 43.31±9.16 | 0.153±0.02 |
| Model group | 0 | - | - |
| Acyclovir treatment group | 6 | 79.21±6.23 | 0.264±0.01 |
| Composition treatment group | 10 | 94.11±9.31 | 0.172±0.03 |

This experiment shows that the composition can significantly reduce the mortality of mice with herpes virus infection, increase the release of NO in mice in the infection process, and reduce the level of inflammatory factors.

### Embodiment 15 Screening of antiseptic formula of 5-methyltetrahydrofolic acid composition

6-8-week-old male C57 mice weighing 18-22 g were used. The general physiological index, body weight and food intake conditions of the mice were observed. The mice were adaptively fed for one week with standard pellet feed and free drinking water, under natural day and night lighting at a room temperature of 18-26°C and relative humidity of 40%-70%. LPS was purchased from sigma company, with an article number of L2880.

49 male C57 mice were divided into 7 groups, including 6 administration groups and 1 model group, 7 mice in each group. Each group was intraperitoneally injected with LPS as per 13 mg/kg (the dose was determined by a pre-experiment, because the 120-hour condition cannot be observed under an LPS dose of 20 mg/kg, to prolong the survival time of the model group, the pre-experiment confirmed that the dose is 13 mg/kg).
Group A, 5-methyltetrahydrofolate calcium: vitamin C=3:1 (equivalent to a dosage of 300 mg of 5-methyltetrahydrofolate calcium and 100 mg of vitamin C for human);
Group B, 5-methyltetrahydrofolate calcium: vitamin C=1:3 (equivalent to a dosage of 100 mg of 5-methyltetrahydrofolate calcium and 300 mg of vitamin C for human);
Group C, 5-methyltetrahydrofolate calcium: vitamin C=1:12 (equivalent to a dosage of 50 mg of 5-methyltetrahydrofolate calcium and 600 mg of vitamin C for human);
Group D, 5-methyltetrahydrofolate calcium: vitamin C=12:1 (equivalent to a dosage of 600 mg of 5-methyltetrahydrofolate calcium and 50 mg of vitamin C for human);
Group E, 5-methyltetrahydrofolate calcium: vitamin C=4:1 (equivalent to a dosage of 1200 mg of 5-methyltetrahydrofolate calcium and 300 mg of vitamin C for human);
Group F, 5-methyltetrahydrofolate calcium: vitamin C=3:1 (equivalent to a dosage of 1200 mg of 5-methyltetrahydrofolate calcium and 400 mg of vitamin C for human);
Group H, model group.

All the administration groups were given 3 doses: one dose was given 9 hours after the model was made (9 o'clock in the evening on the first day), one dose was given in the morning of the next day, and one dose was given in the morning of the third day, for a total of 3 times, with the same dosage volume. The results are as follows.

**Table 6 Animal performance and death at each time point after intraperitoneal injection of LPS**

| Groups | Death time | | | | 24 h survival rate | 48 h survival rate | 72 h survival rate | 144 h survival rate |
|---|---|---|---|---|---|---|---|---|
| | 24 h | 48 h | 72 h | 144 h | | | | |
| Group A | | 1 | 1 | 1 | 100.00% | 85.71% | 71.43% | 57.14% |
| Group B | | 1 | 1 | 2 | 100.00% | 85.71% | 71.43% | 42.86% |
| Group C | | 1 | 2 | 1 | 100.00% | 85.71% | 57.14% | 42.86% |
| Group D | | 2 | 2 | 1 | 71.43% | 42.85% | 28.57% | 28.57% |
| Group E | | | | 1 | 100.00% | 100.00% | 85.71% | 85.71% |
| Group F | | | | | 100.00% | 100.00% | 100.00% | 100.00% |
| Model group | | | 4 | 1 | 100.00% | 100.00% | 42.86% | 28.57% |

During the administration, some mice in groups C and D showed signs of trembling and obvious listlessness. Groups E and F were in the best condition, followed by groups A and B. No animals died in all groups after 144 h.

The results show that different dosages of the composition of 5-methyltetrahydrofolic acid and vitamin C in different ratios could inhibit the mortality of mice induced by LPS to varying degrees and improve the survival rate of the mice. Oral gavage could achieve a good effect of reducing the mortality of sepsis model mice, and the best ratio was 3:1. The effect was significantly improved with the increase of the dose. 1200 mg of 5-methyltetrahydrofolic acid equivalent to the dose for human, can interact with 400 mg of vitamin C to survive 100% of the LPS-induced sepsis model mice, which has great clinical value.

### Embodiment 16 Attempt to protect model mice with Staphylococcus aureus-induced sepsis by the 5-methyltetrahydrofolic acid composition

SPF-grade Kunming mice weighing about 20 g were used. A single colony of *Staphylococcus aureus* was inoculated into a culture solution, and shaking was performed for culture overnight at 37°C. The bacterial solution was collected and centrifuged at 4000 rpm for 3 min, and the precipitate was collected and washed twice with sterile normal saline. The bacterial solution was about 5×10⁹ CFU/ml (pre-experiments showed that intraperitoneal injection of 2 ml of bacterial solution had a 7-day mortality rate of 90% or above).

The mice were randomly divided into 5 groups, half male and half female, and grouped as follows:
Group A, high-dose group, 5-methyltetrahydrofolate calcium: vitamin C=3:1 (equivalent to a dosage of 1200 mg/day or 192 mg/kg/day for human);
Group B, medium-dose group, 5-methyltetrahydrofolate calcium: vitamin C=3:1 (equivalent to a dosage of 600 mg/day or 96 mg/kg/day for human);
Group C, low-dose group, 5-methyltetrahydrofolate calcium: vitamin C=3:1 (equivalent to a dosage of 300 mg/day or 48 mg/kg/day for human);
Group D, combined treatment, 5-methyltetrahydrofolate calcium: vitamin C=3:1 (equivalent to a dosage of 600 mg/day or 96 mg/kg/day for human) + oxacillin 30 mg/kg/d;

Model group. 4 h after the intraperitoneal injection of the bacterial solution, the mice were administered according to the above dose three times (on day 0, day 2, and day 4), once every other day.

**Table 7 Treatment of Staphylococcus aureus-induced sepsis model**

| Groups | Death time | | | | | | | | Survival rate |
|---|---|---|---|---|---|---|---|---|---|
| | 0 | 1 | 2 | 3 | 4 | 5 | 6 | 7 | |
| Group A | 3 | 4 | 2 | | | | | | 10% |
| Group B | 3 | 2 | 1 | 2 | | | 1 | | 10% |
| Group C | 2 | 1 | 2 | 1 | 2 | 2 | | | 0% |
| Group D | 1 | 2 | | 1 | 1 | 2 | 1 | 1 | 10% |
| Model group | 2 | | 2 | 1 | 2 | 1 | 1 | 1 | 0% |

The results of the experiment are surprising. The composition with an accurate protective effect on the LPS model mice cannot alleviate the disease of the *Staphylococcus* model mice. There is no significant difference between the two types of model mice.

### Embodiment 17 Treatment of sepsis and antibacterial experiment with the composition of formula C

5-methyltetrahydrofolate calcium, arginine, and phytohemagglutinin were mixed in a ratio of 2:8:1 to obtain a composition of formula C.

120 healthy ICR mice weighing 18-24 g, half male and half female were used. *Staphylococcus aureus* and *Streptococcus pneumoniae* were used as the test bacteria, and the mice were randomly divided into a normal group, a model group, a low-dose composition group (40 mg/kg), a medium-dose composition group (80 mg/kg), a high-dose composition group (160 mg/kg), and an amoxicillin group (120 mg/kg) according to body weight. The above groups were administered intraperitoneally as per 20 ml/kg once a day. Except the normal group, mice in the other groups were intraperitoneally injected with a *Staphylococcus aureus* solution (5×10⁹ CFU/ml) as per 0.5 ml/mouse. (*Streptococcus pneumoniae* infection method and grouping were the same as those of *Staphylococcus aureus*) The death of mice in each group within 4 days after injection of the bacterial solution was observed, the differences among the groups were compared, and the survival rate was calculated. The experimental results are as follows.

**Table 8 The tested composition has a protective effect on infected mice (x̅±s, n=10)**

| Groups | Dose mg/kg | Survival rate (%) | |
|---|---|---|---|
| | | *Staphylococcus aureus* | *Streptococcus pneumoniae* |
| Normal group | - | 100 | 100 |
| Model group | - | 10 | 10 |
| Amoxicillin group | 120 | 70^{∗∗} | 50^{∗} |
| Low-dose composition group | 40 | 20 | 20 |
| Medium-dose composition group | 80 | 40^{∗} | 30^{∗} |
| High-dose composition group | 160 | 70^{∗∗} | 60^{∗∗} |

| | | | |
|---|---|---|---|
| Note: Compared with the model control group, ^{∗}p<0.05; compared with the model control group, ^{∗∗}p<0.01 | | | |

The results show that for model animals with clear infection, L-arginine should be added to the composition.

The composition could significantly improve the survival rate of the host. To further verify the effect of the composition on lymphocytes, an independent experiment was carried out. ICR mice were injected with 0.5 ml of *Staphylococcus aureus* solution (5×10⁹ CFU/ml) respectively, among which 10 model mice were injected intraperitoneally with a composition solution of 5-methyltetrahydrofolic acid and arginine as per 20 mg/kg. 24 hours after modeling, the mice were sacrificed and spleens were collected. The spleens were subjected to total cell counting and splenic lymphocyte staining analysis (surface staining, B cells, CD4⁺ T cells, CD8⁺ T cells, and NK cells).

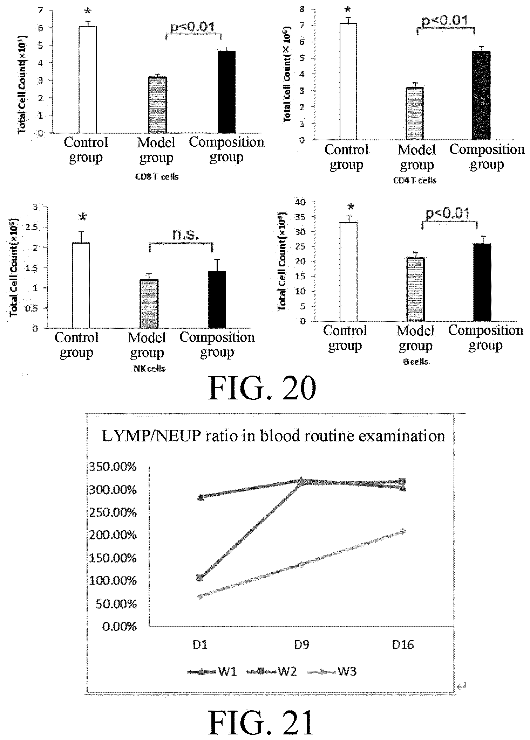

The results are shown in FIG. 20, and the results show that the composition could prevent apoptosis of CD4 and CD8 T cells in sepsis. In the septic mice 24 hours after modeling, sepsis induced apoptosis of all types of immune effector cells. The composition prevented the apoptosis of CD4 T and CD8 T cells and B cells, but did not prevent the decrease of NK cells (n=11). This is an effect of the composition which has not been reported so far, and the prospect of the composition in the treatment of sepsis should be fully considered.

### Embodiment 18 Inhibitory effect of the combination on gram-negative bacteria in vivo

5-methyltetrahydrofolate calcium, arginine, and phytohemagglutinin were mixed in a ratio of 2:8:1, and after total mixing, a composition of formula C was prepared.

50 BAL B/C male mice weighing 18-22 g were divided into 6 groups with 8 mice in each group. Among them, 2 groups were experimental groups, and the rest were control groups, namely a low-dose composition group (40 mg/kg), a high-dose composition group (80 mg/kg), a normal group, a model group, a penicillin group (450 mg/kg), and a meropenem group (75 mg/kg) respectively. After a bacterial solution of *Pseudomonas aeruginosa* was cultured by streaking on an LB solid medium, a typical colony was picked and inoculated into an ordinary LB liquid medium. After overnight shaking culture at 37°C for about 12 h, the culture was centrifuged at 4000 r/min for 3 min, the supernatant was discarded, and the bacteria were resuspended in normal saline for later use. The BAL B/C male mice in each experimental group and three control groups were intraperitoneally injected with a fatal dose of *Pseudomonas aeruginosa* solution at a dose of 500 µL/mouse. 30 min after the BAL B/C male mice in the experimental groups were infected with the bacteria, the medium-dose composition group and the high-dose composition group were administered with drugs by gavage, the penicillin group and the meropenem group were also administered with drugs by gavage, and the normal group was administered with purified water by gavage. 24 h after administration, the BAL B/C male mice in each experimental group and control group were administered for the second time, and the drug type and dosage were the same as the administration for the first time. The BAL B/C male mice were observed every 24 h after administration, and their survival was recorded. All animals were sacrificed on the day 15.

The results show that the composition of formula C of the present invention could inhibit Gram-negative bacteria in animals and had a low toxicity. The composition of the present invention could maintain 100% survival of mice infected with the fatal dose of *Pseudomonas aeruginosa* in 14 days. The survival rate of mice in the meropenem group was 100% after 14 days, while all the mice in the penicillin group died, and all the mice in the model group died.

### Embodiment 19 Death protection effect of treatment and preventive administration with the composition on mice infected with H1N1 (FM1) influenza virus

### 1.1 Tested sample

Composition granules (5-methyltetrahydrofolate calcium:arginine=1:4), Lianyungang Jinkang Hexin Pharmaceutical Co., Ltd.

### 1.2 Experimental animals

SPF-grade ICR mice weighing 13-15 g, half male and half female, provided by Beijing Vital River Laboratory Animal Co., Ltd., with a license number of SCXK (Beijing) 2016-0006, and an animal certificate of 1100111911082385.

### 1.3 Toxic strains for inoculation

FM1 toxic strains (at a concentration of 100. TICD₅₀) were purchased, passaged by the inventor's laboratory, and stored in a refrigerator at -80°C.

### 2 Experimental methods and results

### 2.1 Dosage design

In the experiment, the test animals were all subjected to nasal drip infection with a 1000-fold dilution of the FM1 toxic strain, and the composition was divided into three dosage groups of high, medium and low. In addition, a preventive administration group was set up, and administered at a low dose once. A vitamin group was set up to compare with the middle-dose group.

### 2.2 Bacterial solution preparation

0.2 ml of the FM1 toxic strains were freeze-thawed and subjected to gradient dilution with normal saline to obtain the required concentration (1000-fold) for the experiment.

### 3.3 Determination of infective dose of animals

Mice were infected with FM1 virus solutions of different concentrations by nasal drip as per 45 µl/mouse. There were 10 mice in each concentration group. The death of animals within 12 days after infection was observed, and the concentration of the virus solution that caused 80±5% of the death of mice was used as the infective concentration for the formal experiment. The results are shown in Table 9.

**Table 9 Determination of the concentration of a solution of influenza virus FM1 causing death in mice**

| Concentration | Number of animals | Number of deaths | Mortality (%) |
|---|---|---|---|
| 250-fold diluent | 10 | 10 | 100 |
| 500-fold diluent | 10 | 9 | 90 |
| 1000-fold diluent | 10 | 8 | 80 |

According to the above results, the 1000-fold FM1 diluent was used in the formal experiment as per 45 µl/mouse by nasal drip infection.

### 4.1 Animal infection and grouping

A total of 130 ICR mice were randomly divided into 7 groups according to body weight levels, namely, a normal control group, a model control group, high-, medium-, and low-dose composition groups, a composition preventive group, and a composition post-treatment group. Except 10 mice in the normal control group, 20 mice in each of the other groups were infected with H1N1 influenza virus by nasal drip as per 45 µl/mouse. After infection, each administration group was administered by gavage as per 0.1 ml/10 g.

### 4.2 Dosage design for therapeutic administration and preventive administration

The normal control group was given the same volume of normal saline.

The model control group was given the same volume of model saline.

The high-dose group was administered with the composition (5-methyltetrahydrofolate calcium: arginine=1:4) as per 0.346 g/kg body weight twice, the administration time was 12 h and 24 h after infection respectively, and the second administration dose was half of the first dose.

The medium-dose group was administered with the composition (5-methyltetrahydrofolate calcium: arginine=1:4) as per 0.173 g/kg body weight twice, the administration time was 12 h and 24 h after infection respectively, and the second administration dose was half of the first dose.

The low-dose group was administered with the composition (5-methyltetrahydrofolate calcium: arginine=1:4) as per 0.087 g/kg body weight twice, the administration time was 12 h and 24 h after infection respectively, and the second administration dose was half of the first dose.

The preventive group for a preventive effect was given the dose of the low-dose group once 12 h before modeling, that is, the composition (5-methyltetrahydrofolate calcium: arginine=1:4) as per 0.087 g/kg body weight.

The post-treatment group was administered with the composition (5-methyltetrahydrofolic acid: arginine=1:4) as per 0.173 g/kg body weight twice, the administration time was 12 h and 24 h after infection respectively, and the second administration dose was half of the first dose. The composition (5-methyltetrahydrofolic acid: arginine: vitamin C = 3:12:1) was administered as per 0.173 g/kg body weight respectively on day 3 and day 6 after infection.

The death of animals was observed within 14 days after infection, and the mortality and death protection rate ((control group mortality rate-experimental group mortality rate)/control group mortality rate) were calculated. Lung index = wet lung weight (g)/body weight (g). The results were statistically processed by X2 test and t test for comparison among groups. The results are shown in the table below.

**Table 10 Protective effects on death of mice caused by primary infection with FM1 influenza virus**

| Groups | Number of animals | Number of deaths | Mortality (%) | Protective rate (%) |
|---|---|---|---|---|
| Normal control group | 10 | 0 | 0 | |
| Model control group | 20 | 14 | 70 | - |
| High-dose group | 20 | 3 | 15 | 78.57 |
| Medium-dose group | 20 | 6 | 30 | 57.14 |
| Low-dose group | 20 | 8 | 40 | 42.86 |
| Preventive group | 20 | 11 | 55 | 21.43 |
| Post-treatment group | 20 | 2 | 10 | 85.71 |

**Table 11 Effects of FM1 influenza virus on lung inflammation in mice**

| Groups | Number of animals | Lung index |
|---|---|---|
| Normal control group | 10 | 0.54 |
| Model control group | 3 | 1.20 |
| High-dose group | 17 | 0.86 |
| Medium-dose group | 14 | 0.95 |
| Low-dose group | 11 | 1.10 |
| Preventive group | 8 | 1.14 |
| Post-treatment group | 18 | 0.85 |

### 5. Death protective effects of the composition on repeated infections in surviving mice after treatment

In the above experiment, on day 15 after administration, the mice which survived the above influenza virus infection or survived after drug intervention will be subjected to a repeated infection experiment. The surviving mice were infected with the same kind of influenza virus once again, and the deaths within 7 days of reinfection were observed. The effects of different treatment groups on the mortality and death protection rate of mice reinfected with the influenza virus were compared. No drug intervention was conducted in each group in the repeated infection experiment.

**Table 12 Protective effect of the composition on the death of mice reinfected with FM1 influenza virus**

| Groups | Number of reinfected mice | Number of deaths of reinfection | Mortality of reinfection (%) | Protective rate of reinfection (%) |
|---|---|---|---|---|
| Normal control group | 10 | 0 | 0 | - |
| Model control group | 6 | 3 | 50 | - |
| High-dose group | 17 | 0 | 0 | 100 |
| Medium-dose group | 14 | 0 | 0 | 100 |
| Low-dose group | 12 | 1 | 8.33 | 83.34 |
| Preventive group | 9 | 1 | 11.11 | 77.78 |
| Post-treatment group | 18 | 0 | 0 | 100 |

The results show that the high-dose composition group had a protective effect on animal death after the primary infection and repeated infection. The preventive administration group also had a certain protective effect, which suggests that in addition to reduction of the mortality of mice caused by influenza virus, and better therapeutic and death protection effects on animals, preventive administration of the composition also showed a certain protective effect, and could prolong the survival time of mice.

### Embodiment 20 Inhibitory effects of a composition of formula A on fever caused by endotoxin

Preparation of a composition of formula A: 5-methyltetrahydrofolate calcium and vitamin C were mixed at a mass ratio of 1:1, and after three-dimensional mixing and total mixing, the composition of formula A was prepared.

Endotoxin preparation: According to previous reports, the pyrogenic dose of endotoxin was determined to be 250 ng/ml/kg after a pre-experiment, and endotoxin was prepared with normal saline before the experiment.

Selection of rabbits: 35 New Zealand rabbits weighing 2.0-3.0 kg were selected, and measured for rectal temperature once a day for 2 consecutive days to adapt the rabbits to the temperature measurement operation. Rabbits whose body temperature was in a range of 37.5-38.5°C and had a body temperature fluctuation within 0.5°C were selected for the experiment.

Each rabbit was injected with endotoxin from the ear vein. One hour after the injection, the rectal temperature was measured, and the rabbits were divided into groups equally according to changes in body temperature, namely, a model group, a positive drug group, and high- (40 mg/kg), medium- (20 mg/kg), and low- (10 mg/kg) dose groups of the pharmaceutical composition of formula A of the present invention. Each administration group was administered once by gavage as per 2 ml/kg, and the model group was given distilled water under the same conditions. The rectal temperature was measured 0.5 h, 1 h, 1.5 h, and 2 h respectively after administration. The experimental results are shown in Table 13.

**Table 13 Effect of the composition of formula A of the present invention on body temperature changes of rabbits with endotoxin-induced fever (n=6)**

| Groups | Basal body temperature | Body temperature after molding | Body temperature after administration (°C) (x̅±s) | | | |
|---|---|---|---|---|---|---|
| | | | 0.5h | 1 h | 1.5 h | 2 h |
| Model group | 37.5±0.2 | 39.0±0.3 | 39.0±0.3 | 39.1±0.4 | 39.0±0.4 | 38.9±0.3 |
| Shuanghuanglian | 37.6±0.3 | 38.9±0.3 | 38.6±0.4 | 38.6±0.4 | 38.5±0.4 | 38.3±0.3 |
| High dose | 37.5±0.3 | 39.1±0.3 | 38.6±0.5 | 38.5±0.4 | 38.4±0.4 | 38.5±0.2 |
| Medium dose | 37.4±0.2 | 39.0±0.2 | 38.7±0.3 | 38.8±0.4 | 38.7±0.3 | 38.6±0.3 |
| Low dose | 37.5±0.3 | 38.9±0.3 | 38.7±0.3 | 38.7±0.4 | 38.7±0.4 | 38.7±0.3 |

The results showed that the body temperature of the rabbits in each group increased significantly after the injection of endotoxin, and the body temperature of the rabbits in each group decreased after the administration, which indicates that the composition has a certain antipyretic effect.

### Embodiment 21 In vivo anti-mycoplasma pneumonia experiment with formula C

The international standard strain of mycoplasma pneumoniae (ATCCFH15531) was purchased from American Type Culture Collection.

A composition of formula C was prepared in the laboratory. 5-methyltetrahydrofolate calcium, arginine, and phytohemagglutinin were mixed in a ratio of 2:8:1, and after total mixing, the composition of formula C was obtained.

50 BALB/C mice, half male and half female, weighing 16-20 g, were purchased from Guangdong Medical Experimental Animal Center.

A positive drug group was administered with azithromycin dispersible tablets, produced by Harbin Pharmaceutical Group No. 6 Pharmaceutical Factory, with a batch number of 160303, and a specification of 0.25 g/tablet.

After one week of adaptive feeding, BALB/C mice were randomly divided into 5 groups, half male and half female, namely a normal control group, a model control group, a positive drug control group (40 mg/kg), a high-dose composition C group (80 mg/kg), and a low-dose composition C group (40 mg/kg). Except the normal control group, the mice in the other groups were anesthetized with ether, and were infected with 50 µL of mycoplasma pneumoniae (MP) bacterial solution with an infective concent of 10⁶ CCU/ml by nasal drip for 3 consecutive days. After that, drugs were administered by gavage once a day for 10 consecutive days. 4 h after the last administration, blood was collected from the mice's eyeballs and the mice were sacrificed. The lungs, spleen, and thymus were weighed and subjected to pathological observation. A small piece of lung tissue was taken and ground to quantitatively detect the content of MP by PCR. The results are as follows.

**Table 14 Effects on splenic index and thymic index of mice**

| Groups | Administration dose | Splenic index (mg/g) | Thymic index (mg/g) |
|---|---|---|---|
| Normal control group | - | 4.445±0.876^{∗} | 3.212±1.342 |
| Model control group | - | 6.163±2.047 | 3.253±1.164 |
| Positive drug control group | 40 | 4.515±0.982^{∗} | 3.818±1.232 |
| High-dose composition C group | 80 | 4.297±0.844^{∗} | 3.273±1.362 |
| Low-dose composition C group | 40 | 4.684±0.931^{∗} | 3.311±1.171 |

| | | | |
|---|---|---|---|
| Note: Compared with the model control group, ^{∗}p<0.05 | | | |

Compared with the model group, the splenic index of the mice in the blank control group was significantly different, and the splenic index of the mice in each administration group was significantly different, which suggests that MP was killed in the body after the administration. Stimulation on immune organs was reduced and the splenic index was reduced.

Histopathological examination of the lung tissue of mice showed that the lung tissue of the model group had obvious pathological changes compared with the normal group during dissection, the lungs appeared to be congested and edematous, and there were unequal necrotic foci in the lung lobes. Pathological examination showed that the pathological changes were mainly in the lungs, mainly interstitial pneumonia and bronchiolar pneumonia, with obvious lymphocytic infiltration in the bronchus. The lung tissue of the blank group was basically normal. Mild interstitial pneumonia could be seen in the azithromycin control group. Inflammation in the composition C group was significantly reduced, slight inflammatory cell infiltration could be seen around the bronchioles, and the degree of interstitial pneumonia gradually decreased with the increase of the dose. The results show that the composition C had an effect of controlling infection of mycoplasma pneumoniae in mice, and alleviated pathological changes in the lung tissue.

### Embodiment 22 Influence of nitric oxide composition as immune adjuvant on effect of rabies virus vaccine

30 adult Kunming mice weighing 20-28 g, half male and half female were purchased from the Experimental Animal Center of Xinjiang Medical University. Rabies rSRV9 live attenuated oral freeze-dried vaccines were purchased from Beijing United Health Biotechnology Co., Ltd. A nitric oxide composition of formula B was prepared by mixing 5-methyltetrahydrofolate calcium and arginine at a mass ratio of 1:4 to obtain the composition of formula B. The 30 mice, half male and half female, were divided into 3 groups with 10 mice in each group, namely a blank control group, a virus oral immunization group, and a formula B + virus oral immunization group. Corresponding vaccines were taken orally on days 1, 7, and 14 respectively, and blood was collected from the orbit as per 300 µL/mouse on days 0, 14, 21, 35, 42, and 70 after immunization. After standing for 1 h, the blood was centrifuged at 5000 r/min for 5 min, and serum was pipetted. Simultaneously, about 0.05 g of mouse feces was collected, put in 500 µL of PBS (with a pH value of about 7.4), and pulverized to form a turbid solution; the turbid solution was centrifuged to pipet the supernatant; and the supernatant was stored in a refrigerator at -20°C. Serum IgG antibodies were detected by an ELISA detection kit, and fecal IgA antibodies were detected by a mouse serum rabies-specific IgA antibody ELISA detection kit. The results are shown in the table below.

**Table 15 Serum anti-rabies-specific IgG levels (U/ml) detected at different times after initial immunization of mice in each group**

| Groups | 14 d | 21 d | 35 d | 42 d | 70 d |
|---|---|---|---|---|---|
| Blank control group | 73.00±53.26 | 73.42±55.73 | 71.13±52.73 | 79.73±51.72 | 75.32±54.61 |
| Composition B + oral vaccine group | 198.15±63.82 | 267.35±61.63 | 896.14±83.85 | 1945.76±94.44 | 2693.79±75.23 |
| Oral vaccine group | 105.93±55.84 | 200.63±61.24 | 492.53±62.12 | 1013.83±59.43 | 1893.25±74.91 |

The results show that the composition B could increase the level of IgG antibodies in the serum. After the oral vaccine was combined with the composition B, there was a considerable degree of antibodies on day 14. 21 days after immunization, there were significant differences in antibodies among different groups.

**Table 16 Fecal SIgA levels (U/ml) detected at different times after initial immunization of mice in each group**

| Groups | 14 d | 21 d | 35 d | 42 d | 70 d |
|---|---|---|---|---|---|
| Blank control group | 1.63±0.01 | 0.74±0.08 | 0.22±0.08 | 0.32±0.08 | 0.92±0.09 |
| Composition B + oral vaccine group | 24.22±0.33 | 35.22±0.27 | 42.43±0.35 | 51.03+1.28 | 62.01+2.14 |
| Oral vaccine group | 20.35±0.24 | 31.25±0.16 | 35.35±0.4.4 | 43.21±0.56 | 47.53±0.41 |

The above results show that the composition of formula B could improve the immunological competence of the vaccine. Under a synergistic effect of the composition of formula B as an immune adjuvant, the rSRV9 virus oral attenuated vaccine could significantly increase antibody expression in mice, and had the actions of reducing the number of immunizations, and improving the immune effect.

### Embodiment 23 Use of the composition of formula C in treatment of African swine fever

A case of African swine fever was confirmed by the China Animal Health and Epidemiology Research Center on a sample sent by Jiangsu Animal Disease Prevention and Control Center. The positive sample came from a farmer in Ganyu District, Lianyungang, Jiangsu Province. The farmer had 300 live pigs, 130 cases of disease, and more than 120 deaths. The dead sick pigs were dissected, and pathological examination found pulmonary hemorrhage, interstitial pneumonia and other symptoms; spleen dissection revealed severe splenomegaly by 7 times; stomach dissection revealed diffuse hemorrhage on the gastric serosal surface; and kidney swelling was obvious, which was in line with the symptoms of African swine fever.

Blood of 3 sick pigs and 10 healthy pigs from the farmer were collected and centrifuged at 3000 r/min, the serum was added to Roche with ceramic beads, and a PBS buffer was added. DNA was extracted using a viral DNA kit, and the virus was determined to be ASFV African swine fever gene type II, which belongs to the virus genus broadcast in the Russian Far East and Eastern Europe in 2017. The composition of formula C was used to intervene in treatment of African swine fever.

Preparation of a composition injection of formula C: 5-methyltetrahydrofolate calcium, L-arginine and phytohemagglutinin were mixed in a ratio of 2:8:1, and after total mixing, the composition of formula C was obtained. The composition of formula C was sterilized, dissolved in normal saline, filtered through a microfiltration membrane, adsorbed with activated carbon to remove a heat source, and then prepared into an injection.

18 pigs at the initial stage of disease were isolated, and harmless treatment of related feed, waste water and feces was conducted. The average body temperature of the sick pigs was 40°C. Some sick pigs had dermohemia and cyanosis, and had multiple bleeding or red spots on the ears and under the abdomen. All sick pigs did not eat normally and lost appetite. Blood tests of the sick pigs found that the level of white blood cells was lower than that of normal pigs.

According to the above situation, the 18 sick pigs were subjected to interventional treatment with the composition of formula C. Each pig was injected with an injection containing the composition of formula C every day at a dose of 50 mg/kg for 2 days, during which the body temperature of each pig was measured.

The results are as follows:

**Table 17 Statistical summary of 7-day survival rate in 18 sick pigs**

| Object | Dose | Number of deaths | Number of survivals | Survival rate (%) |
|---|---|---|---|---|
| Sick pigs with African swine fever | 50 mg/kg | 1 | 17 | 94.4 |

The results show that an unexpected effect was achieved on African swine fever pigs, which suggests that the composition of formula C has a very good antiviral effect. The results show that only one pig died within 7 days. Afterwards, due to policy requirements, all infected pigs were put to death, and other infected pigs of the farmer died 3-4 days after the onset of illness.

### Dissection and detection of death cases of African swine fever

The dead sick pigs were dissected, and it was found that the spleen of the dead sick pigs was severely swollen, and the spleen was congested and fragile; hemorrhage in the lungs, that was, massive hemorrhage in the lungs, were found, and the lung tissue was observed and identified as interstitial pneumonia; hemorrhage was also found in the stomach, with diffuse hemorrhage on the gastric serosal surface; and the kidneys were obviously swollen.

Blood was collected, allowed to stand for 1 h, and centrifuged at 5000 r/min for 5 min, and the serum was pipetted. The level of IgG antibodies was detected. A Krebs-HEPES buffer was added to the blood, and a resulting mixture was allowed to stand at 37°C for 30 min. L-NAME (100 µM) was added, and the contents of superoxide, nitrite and NO were detected by electrochemical methods.

The cured pigs were sacrificed and dissected to conduct pathological observation. It was found that the cured pigs had local hemorrhage in the lungs besides the slightly larger spleen. Blood was collected, allowed to stand for 1 h, and centrifuged at 5000 r/min for 5 min, and the serum was pipetted. The level of IgG antibodies was detected. A Krebs-HEPES buffer was added to the blood, and a resulting mixture was allowed to stand at 37°C for 30 min. L-NAME (100 µM) was added, and the contents of nitrite and NO were detected by photochemical methods.

The results are as follows:

**Table 18 Biochemical indexes of dead sick pigs and cured pigs**

| Groups | IgG (U/ml) | Nitrite (RLU/ml) | NO (RLU/ml) |
|---|---|---|---|
| Dead sick pigs | 1173.00 | 8.5 | 61.1 |
| Cured pigs | 2198.15 | 1.3 | 54.7 |

The results show that there were a large number of antibodies in the cured pigs, which suggests that the composition C can improve the level of a pig's immune system to achieve an anti-viral effect.

It was also found that the level of NO in the dead sick pigs was not low, but the level of RNS greatly increased, which suggested that acute symptoms and death caused by viruses were related to the level of RNS. A high-intensity immune system accelerates the death of individuals, which is common in the course of virus treatment. For some viruses, the survival time of immune knockout mice is much longer than that of normal mice. Therefore, we speculated that the death of African swine fever was related to overreaction of the immune system. Through comparison, it was found that the ratio of RNS/NO in the blood of the dead sick pigs was 3 times as much as that of the cured pigs.

It is suggested that the composition of the present invention can reduce death caused by malignant virus due to immune overexpression by reducing RNS, and maintain normal operation of the immune system to achieve the purpose of clearing the virus. The implementation effect of the composition of the present invention greatly exceeds expectations.

Example 24 Effect of the composition on immune cells of domestic pigs 3 common domestic pigs, about 3 months old, weighing about 25 kg, were administered with a composition containing 5-methyltetrahydrofolic acid. A preparation process of the composition was as follows: 5-methyltetrahydrofolate calcium, L-arginine and phytohemagglutinin were mixed at a ratio of 1:4:0.1 to obtain a pharmaceutical composition. Before the composition was taken, ear blood was collected for blood routine examination. Then, the composition was taken orally as per 30 mg per kilogram of pig body weight every day, and ear blood was collected in the first week and the second week for blood routine examination. The main indexes of the blood routine examination are LYMP (lymphocytes) and NEUP (neutrophils).

The blood routine examination indexes are as follows:

| Days | D1 | | D9 | | D16 | |
|---|---|---|---|---|---|---|
| Pig blood routine examination | LYMP | NEUP | LYMP | NEUP | LYMP | NEUP |
| W1 | 68.7 | 24.2 | 70.1 | 21.8 | 70.7 | 23.2 |
| W2 | 49.0 | 46.7 | 69.5 | 22.2 | 66.3 | 20.9 |
| W3 | 37.8 | 57.6 | 53.9 | 39.6 | 62.1 | 29.8 |

A patient with a higher immune index LYMP/NEUP will have milder symptoms of virus infection. According to clinical analysis of patients with COVID-19 in the article [Zhang B, Zhou X, Qiu Y, et al. Clinical characteristics of 82 death cases with COVID-19[J]. MedRxiv, 2020.], most death cases had a low lymphocyte/neutrophil ratio. In FIG. 21, it is shown that the composition could increase the LYMP/NEUP ratio and could limit the severity of symptoms of virus infection. To a certain extent, it is shown that the composition has a preventive effect on viruses.

The implementations of the present invention are described above. However, the present invention is not limited to the foregoing implementations. Any modification, equivalent replacement, or improvement within the spirit and principle of the present invention should fall within the protection scope of the present invention.

## Claims

1. A pharmaceutical composition for producing a safe amount of nitric oxide in an animal body, comprising an NO toxicity decreasing agent and an optional NO extender, wherein the NO toxicity decreasing agent is selected from antioxidant substances for scavenging peroxynitrous acid or salt thereof (PON) at a dose; preferably, the toxicity decreasing agent does not inhibit expression of inducible nitric oxide synthase (iNOS) at a concentration of not less than 10 µmol/L, for example, does not inhibit expression of iNOS in macrophages induced by LSP.

2. The pharmaceutical composition according to claim 1, wherein the NO toxicity decreasing agent is selected from one or more of the following substances: 5-methyltetrahydrofolic acid or salt thereof, dehydroascorbic acid and NMN.

3. The pharmaceutical composition according to claim 1, wherein the NO extender is selected from enzyme-producing NO substrates; for example, the enzyme-producing NO substrates are selected from L-arginine or salt thereof, citrulline or salt thereof, or arginine activator additive.

4. The pharmaceutical composition according to claim 1, comprising 5-methyltetrahydrofolic acid or salt thereof, and arginine or salt thereof; wherein further, the pharmaceutical composition may comprise phytohemagglutinin.

5. The pharmaceutical composition according to claim 1, wherein a single dose of the 5-methyltetrahydrofolic acid is not less than 15 mg, and a single dose of the arginine is not less than 50 mg.

6. The pharmaceutical composition according to claim 1, wherein the composition comprises 5-methyltetrahydrofolic acid or salt thereof, and vitamin C; preferably, a mass ratio of the 5-methyltetrahydrofolic acid to the vitamin C is 2:1 to 5:1.

7. The pharmaceutical composition according to claim 1, comprising active components and pharmaceutically acceptable auxiliary materials; for example, the pharmaceutical preparation is selected from tablets, capsules, granules, injections, topical ointments or sprays.

8. An immune adjuvant, comprising the composition of any one of claims 1-7.

9. Use of the pharmaceutical composition of any one of claims 1-8 for preparing drugs for preventing or treating diseases caused by pathogenic microorganism infections; preferably, the pathogenic microorganism infection is virus infection.

10. The use of the pharmaceutical composition according to claim 9, wherein the pharmaceutical composition can increase the level of T cells in a virus-infected host, especially CD4 and CD8 T cells, and reduce expression of inflammatory factors, thereby being used for anti-viral infection.

11. The use of the pharmaceutical composition according to claim 9, wherein the virus is influenza virus, herpes virus, and coronavirus such as COVID-19.

12. The use of the pharmaceutical composition according to claim 9, wherein the composition is used for preparing a drug for preventing and treating sepsis and systemic inflammatory response syndrome caused by infection.

13. The use of the pharmaceutical composition according to claim 12, wherein the sepsis is caused by *Staphylococcus aureus, Streptococcus pneumoniae, Pseudomonas aeruginosa,* and influenza virus infection.

14. The use of the pharmaceutical composition according to claim 9, wherein the composition is used for preparing a drug for treating systemic inflammatory response syndrome and sepsis caused by non-infectious factors.

15. The use of the pharmaceutical composition according to claim 14, wherein the composition is as claimed in claim 6.
